(19) Europäisches Patentamt
European Patent Office
Office européen des brevets

(11) **EP 4 303 759 A1**

(12) **EUROPEAN PATENT APPLICATION**
published in accordance with Art. 153(4) EPC

(43) Date of publication:
**10.01.2024 Bulletin 2024/02**

(21) Application number: **22762586.0**

(22) Date of filing: **03.03.2022**

(51) International Patent Classification (IPC):
*G06K 9/00* (2022.01)

(52) Cooperative Patent Classification (CPC):
**A61B 5/372; A61B 5/7267**

(86) International application number:
**PCT/CN2022/078970**

(87) International publication number:
**WO 2022/184124 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.03.2021 CN 202110246494**

(71) Applicant: **Tencent Technology (Shenzhen) Company Limited**
**Shenzhen, Guangdong, 518057 (CN)**

(72) Inventors:
• LIU, Luyan
Shenzhen, Guangdong 518057 (CN)
• MA, Kai
Shenzhen, Guangdong 518057 (CN)
• ZHENG, Yefeng
Shenzhen, Guangdong 518057 (CN)

(74) Representative: **AWA Sweden AB**
**Box 45086**
**104 30 Stockholm (SE)**

(54) **PHYSIOLOGICAL ELECTRICAL SIGNAL CLASSIFICATION AND PROCESSING METHOD AND APPARATUS, COMPUTER DEVICE, AND STORAGE MEDIUM**

(57) This application relates to a method and an apparatus for classification processing of an electrophysiological signal, a computer device, and a storage medium, which relate to the field of artificial intelligence technologies. The method includes: acquiring a to-be-classified electrophysiological signal collected by an acquisition device; acquiring a channel association feature, the channel association feature being generated based on spatial locations of multiple acquisition channels on the acquisition device; extracting a time feature from the collected electrophysiological signal, and embedding the channel association feature into the time feature to obtain an embedded feature; and extracting a spatial feature from the obtained embedded feature, and obtaining a classification result of the electrophysiological signal based on the spatial feature.

FIG. 2

**Description**

RELATED APPLICATION

This application claims priority to Chinese Patent Application No. 2021102464941, entitled "METHOD AND APPARATUS FOR CLASSIFICATION PROCESSING OF ELECTROPHYSIOLOGICAL SIGNAL, COMPUTER DEVICE, AND STORAGE MEDIUM" and filed with the National Intellectual Property Administration, PRC on March 5, 2021, which is incorporated herein by reference in its entirety.

FIELD OF THE TECHNOLOGY

This application relates to the field of computer technologies, and in particular, to a method and an apparatus for classification processing of an electrophysiological signal, a computer device, and a storage medium.

BACKGROUND OF THE DISCLOSURE

With development of computer technologies, deep learning technology has shown obvious advantages in the fields of computer vision, speech recognition and natural language processing. Therefore, deep learning technology has been gradually introduced into the classification task of electrophysiological signals by researchers.

In the conventional technologies, the electrophysiological signals are input into a model for classification, and the model outputs classification results of the electrophysiological signals based on time domain and frequency domain features of the electrophysiological signals. However, when processing the electrophysiological signals, the model only considers the time domain and frequency domain features of the electrophysiological signals, and the undiversified feature dimensions easily lead to inaccurate classification results of the electrophysiological signals.

SUMMARY

According to various embodiments of this application, a method and an apparatus for classification processing of an electrophysiological signal, a computer device, and a storage medium are provided.

A method for classification processing of an electrophysiological signal performed by a computer device is provided, the method including:

acquiring a to-be-classified electrophysiological signal collected by an acquisition device;

acquiring a channel association feature, the channel association feature being generated based on spatial locations of multiple acquisition channels on the acquisition device;

extracting a time feature from the collected electrophysiological signal, and embedding the channel association feature into the time feature to obtain an embedded feature; and

extracting a spatial feature from the obtained embedded feature, and obtaining a classification result of the electrophysiological signal based on the spatial feature.

An apparatus for classification processing of an electrophysiological signal is provided, including:

a signal acquisition module, configured to acquire a to-be-classified electrophysiological signal collected by an acquisition device;

a channel association feature acquisition module, configured to acquire a channel association feature , the channel association feature being generated based on spatial locations of multiple acquisition channels on the acquisition device;

a signal processing module, configured to extract a time feature from the collected electrophysiological signal, and embed the channel association feature into the time feature to obtain an embedded feature; and

a signal classification module, configured to extract a spatial feature from the obtained embedded feature, and obtain a classification result of the electrophysiological signal based on the spatial feature.

A method for classification processing of an electrophysiological signal performed by a computer device is provided, including:

acquiring a training electrophysiological signal collected by an acquisition device and a training label of the training electrophysiological signal;

inputting the training electrophysiological signal into an initial electrophysiological signal classification model corresponding to the acquisition device, the initial electrophysiological signal classification model including a channel association feature, the channel association feature being generated based on spatial locations of multiple acquisition channels on the acquisition device;

extracting a time feature from the training electrophysiological signal through the initial electrophysiological signal classification model, and embedding the channel association feature into the time feature to obtain an embedded feature;

extracting a spatial feature from the obtained embedded feature through the initial electrophysiological signal classification model, and obtaining a predicted label of the training electrophysiological signal based on the spatial feature; and

adjusting a model parameter of the initial electrophysiological signal classification model based on a difference between the training label and the predicted label, until a convergence condition is met, to obtain a electrophysiological signal classification model.

An apparatus for classification processing of an electrophysiological signal is provided, including:

a data acquisition module, configured to acquire a training electrophysiological signal collected by an acquisition device and a training label of the training electrophysiological signal;

a data input module, configured to input the training electrophysiological signal into an initial electrophysiological signal classification model corresponding to the acquisition device, the initial electrophysiological signal classification model including a channel association feature, the channel association feature being generated based on spatial locations of multiple acquisition channels on the acquisition device;

a data processing module, configured to extract a time feature from the training electrophysiological signal through the initial electrophysiological signal classification model, and embed the channel association feature into the time feature to obtain an embedded feature;

a label prediction module, configured to extract a spatial feature from the obtained embedded feature through the initial electrophysiological signal classification model, and obtain a predicted label of the training electrophysiological signal based on the spatial feature; and

a reference adjustment module, configured to adjust a model parameter of the initial electrophysiological signal classification model based on a difference between the training label and the predicted label, until a convergence condition is met, to obtain an electrophysiological signal classification model.

A computer device is provided, including a memory and one or more processors, the memory storing computer-readable instructions, the computer-readable instructions, when executed by the one or more processors, causing the one or more processors to perform the steps of the method for classification processing of an electrophysiological signal described above.

One or more non-volatile computer-readable storage media are provided, storing computer-readable instructions, the computer-readable instructions, when executed by one or more processors, causing the one or more processors to perform the steps of the method for classification processing of an electrophysiological signal described above.

A computer program product or computer program is provided, including computer-readable instructions, the computer-readable instructions being stored in a computer-readable storage medium. One or more processors of a computer device read the computer-readable instructions from the computer-readable storage medium, and execute the computer-readable instructions, to cause the computer device to perform the steps of the method for classification processing of an electrophysiological signal described above.

Details of one or more embodiments of this application are provided in the accompany drawings and descriptions below. Other features, objectives, and advantages of this application become obvious with reference to the specification, the accompanying drawings, and the claims.

BRIEF DESCRIPTION OF THE DRAWINGS

To describe the technical solutions in the embodiments of this application more clearly, the following briefly introduces the accompanying drawings required for describing the embodiments. Apparently, the accompanying drawings in the following description show merely some embodiments of this application, and a person of ordinary skill in the art may still derive other drawings from these accompanying drawings without creative efforts.

FIG. 1 is a diagram of an application environment of a method for classification processing of an electrophysiological signal according to an embodiment.

FIG. 2 is a schematic flowchart of a method for classification processing of an electrophysiological signal according to an embodiment.

FIG. 3A is a schematic flowchart of generating a channel association feature according to an embodiment.

FIG. 3B is a schematic structural diagram of a channel region according to an embodiment.

FIG. 4 is a schematic flowchart of generating a channel association feature according to another embodiment.

FIG. 5 is a schematic flowchart of generating a channel association feature according to another embodiment.

FIG. 6 is a schematic flowchart of a method for classification processing of an electrophysiological signal according to another embodiment.

FIG. 7A is a schematic structural diagram of an electroencephalogram (EEG) signal according to an embodiment.

FIG. 7B is a schematic structural diagram of an EEG signal classification model according to an embodiment.

FIG. 8 is a structural block diagram of an apparatus for classification processing of an electrophysiological signal according to an embodiment.

FIG. 9 is a structural block diagram of an apparatus for classification processing of an electrophysiological signal according to another embodiment.

FIG. 10 is a structural block diagram of an apparatus for classification processing of an electrophysiological signal according to another embodiment.

FIG. 11 is a diagram of an internal structure of a computer device according to an embodiment.

FIG. 12 is a diagram of an internal structure of a computer device according to an embodiment.

DESCRIPTION OF EMBODIMENTS

To make objectives, technical solutions, and advantages of this application clearer and more understandable, this application is further described in detail below with reference to the accompanying drawings and the embodiments. It is to be understood that the specific embodiments described herein are only used for explaining this application, and are not used for limiting this application.

AI technology is a comprehensive subject, relating to a wide range of fields, and involving both hardware and software techniques. Basic AI technologies generally include technologies such as a sensor, a dedicated AI chip, cloud computing, distributed storage, a big data processing technology, an operating/interaction system, and electromechanical integration. AI software technologies mainly include fields such as a computer vision technology, a speech processing technology, a natural language processing technology, machine learning/deep learning, and the like.

Machine learning (ML) is a multi-field interdiscipline and relates to a plurality of disciplines such as the probability theory, statistics, the approximation theory, convex analysis, and the algorithm complexity theory. ML specializes in studying how a computer simulates or implements a human learning behavior to acquire new knowledge or skills, and reorganize an existing knowledge structure, so as to keep improving its performance. The ML is the core of the AI, is a basic way to make the computer intelligent, and is applied to various fields of AI. The ML and DL generally comprise technologies such as an artificial neural network, a belief network, reinforcement learning, transfer learning, inductive learning, and learning from demonstrations.

The solutions provided in the embodiments of this application involve technologies such as ML and big data processing of AI, and are specifically described by using the following embodiments.

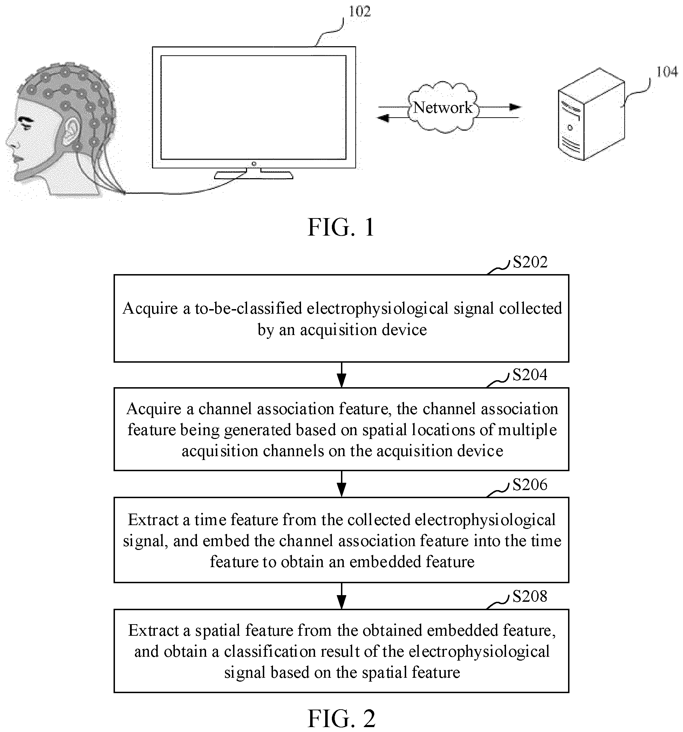

The method for classification processing of an electrophysiological signal provided in this application is applicable to the application environment shown in FIG. 1. A terminal 102 communicates with a server 104 through a network. The terminal 102 may be, but is not limited to, a personal computer, a notebook computer, a smartphone, a tablet computer, an in-vehicle terminal, a portable wearable device, and an electrophysiological signal acquisition device. The server 104 may be implemented by an independent server, a server cluster that includes a plurality of servers, or a cloud server.

Both the terminal 102 and the server 104 may be used independently to perform the method for classification processing of an electrophysiological signal provided in the embodiments of this application.

For example, the terminal acquires a to-be-classified electrophysiological signal collected by an acquisition device, and acquires a channel association feature, where the channel association feature is generated based on spatial locations of multiple acquisition channels on the acquisition device. The terminal can extract a time feature from the collected electrophysiological signal, embed the channel association feature into the time feature to obtain an embedded feature, extract a spatial feature from the obtained embedded feature, and obtain a classification result of the electrophysiological signal based on the spatial feature. The terminal can display the classification result.

The server acquires a training electrophysiological signal collected by an acquisition device and a training label of the training electrophysiological signal. The server inputs the training electrophysiological signal into an initial electrophysiological signal classification model corresponding to the acquisition device, where the initial electrophysiological signal classification model includes a channel association feature, and the channel association feature is generated based on spatial locations of multiple acquisition channels on the acquisition device. The server can extract a time feature from the training electrophysiological signal through the initial electrophysiological signal classification model, embed the channel association feature into the time feature to obtain an embedded feature, extract a spatial feature from the obtained embedded feature through the initial electrophysiological signal classification model, obtain a predicted label of the training electrophysiological signal based on the spatial feature, and adjust a model parameter of the initial electrophysiological signal classification model based on a difference between the training label and the predicted label, until a convergence condition is met, to obtain a electrophysiological signal classification model. The server can also send the electrophysiological signal classification model to the terminal for use.

Both the terminal 102 and the server 104 may be alternatively used in cooperation to perform the method for classification processing of an electrophysiological signal provided in the embodiments of this application.

For example, the server can acquire, from the terminal, a to-be-classified electrophysiological signal collected by an acquisition device. The server classifies the electrophysiological signal to obtain a classification result of the electrophysiological signal. Further, the server may send the classification result to the terminal and the classification result is displayed on the terminal.

The server acquires, from the terminal, a training electrophysiological signal collected by the acquisition device and a training label of the training electrophysiological signal, and trains an initial electrophysiological signal classification model based on the training electrophysiological signal and the corresponding training label to obtain a electrophysiological signal classification model.

In the embodiments of the present disclosure, including the embodiments of the claims and the specification (hereinafter referred to as all embodiments of the present disclosure), as shown in FIG. 2, an exemplary method for classification processing of an electrophysiological signal is provided, which is described by using an example that the method is applied to a computer device shown in FIG. 1. The computer device may be the terminal 102 or the server 104 in FIG. 1. Referring to FIG. 2, the method for classification processing of an electrophysiological signal includes the following steps:

Step S202. Acquire a to-be-classified electrophysiological signal collected by an acquisition device. In some embodiments of the present disclosure, the acquisition device can be preferably configured as the target acquisition device.

The acquisition device refers to a device for collecting electrophysiological signals. The target acquisition device refers to an acquisition device corresponding to the to-be-classified electrophysiological signal. The electrophysiological signal refers to a physiological signal presented by a current or voltage, for reflecting electrophysiological activities of nerve cells. The electrophysiological signal may be an electroencephalogram signal, an electromyography signal, an electrocardiogram signal, or the like.

Specifically, the computer device may acquire the to-be-classified electrophysiological signal collected by the target acquisition device locally, or from another terminal or a server. The to-be-classified electrophysiological signal may be an electrophysiological signal collected in real time. The target acquisition device can collect electrophysiological signals in real time, and the computer device can classify a latest electrophysiological signal in real time to obtain a corresponding classification result. Certainly, the to-be-classified electrophysiological signal may alternatively be an electrophysiological signal collected historically. The electrophysiological signal collected by the target acquisition device in real time can be stored in a database of the terminal or server. The computer device can obtain the electrophysiological signal collected historically from the database, and classify the electrophysiological signal to obtain a corresponding classification result.

Step S204. Acquire a channel association feature, the channel association feature being generated based on spatial locations of multiple acquisition channels on the acquisition device. In some embodiments of the present disclosure, the channel association feature can be preferably configured as the target channel association feature.

The acquisition device includes multiple electrodes, and different electrodes are used for collecting electrophysiological signals at different locations. One electrode corresponds to one acquisition channel. Therefore, the acquisition device includes multiple acquisition channels, and different acquisition channels are used for collecting electrophysiological signals at different locations. Neurons that are close in space are more likely to be physically connected, while neurons that are far away from each other are less likely to be physically connected. Physically connected neurons can be considered as closely linked neurons, and electrophysiological signals generated by closely linked neurons are also closely linked, interrelated and interacting. Therefore, a channel association feature can be generated based on the spatial locations of the multiple acquisition channels on the acquisition device, so that during classification of the electrophysiological signals, data processing and information fusion can be performed on the electrophysiological signals by using the channel association feature, thereby obtaining a more accurate classification result. The channel association feature is feature information for describing associations among acquisition channels. The associations among the acquisition channels may be determined according to the spatial locations of the acquisition channels. Further, locations of acquisition channels are different on different acquisition devices. Therefore, a channel association feature corresponding to each acquisition device needs to be established, and the target channel association feature refers to the channel association feature corresponding to the target acquisition device.

Specifically, the computer device may generate the channel association feature corresponding to the acquisition device based on the spatial locations of the acquisition channels on the acquisition channels. The computer device may pre-generate channel association features corresponding to the acquisition devices respectively. Therefore, after acquiring the to-be-classified electrophysiological signal collected by the target acquisition device, the computer device may directly acquire the pre-generated target channel association feature corresponding to the target acquisition device. Certainly, the computer device can also generate the target channel association feature corresponding to the target acquisition device in real time. Therefore, after acquiring the to-be-classified electrophysiological signal collected by the target acquisition device, the computer device can acquire spatial locations of multiple acquisition channels on the target acquisition device and generate the target channel association feature in real time based on the spatial locations of the multiple acquisition channels on the target acquisition device.

In all embodiments of the present disclosure, the computer device can determine a plurality of spatially close acquisition channels based on spatial location distances between the acquisition channels, associate the spatially close acquisition channels, and generate a channel association feature based on the associated acquisition channels. Two acquisition channels being spatially close specifically means that a spatial distance between the two acquisition channels is less than a distance threshold.

In all embodiments of the present disclosure, in order to improve accuracy of the channel association feature, the computer device can map the spatial locations of the acquisition channels to the same plane to obtain plane locations of the acquisition channels; determine a plurality of closely related acquisition channels based on plane location distances between the acquisition channels, associate the closely related acquisition channels, and generate a channel association feature based on the associated acquisition channels. The closely related acquisition channels may be determined based on a region shape feature of a channel region formed by the acquisition channels.

Step S206. Extract a time feature from the collected electrophysiological signal, and embed the channel association feature into the time feature to obtain an embedded feature. In some embodiments of the present disclosure, the time feature can be preferably configured as the target time feature, and the embedded feature can be preferably configured as the target embedded feature.

Specifically, the computer device may perform time feature extraction on the to-be-classified electrophysiological signal to obtain a target time feature corresponding to the to-be-classified electrophysiological signal, and then embed the target channel association feature into the target time feature to obtain a target embedded feature.

In all embodiments of the present disclosure, the computer device may perform multiple time feature extractions on the to-be-classified electrophysiological signal to obtain multiple intermediate time features, and the target time feature is formed by the intermediate time features. During embedding of the target channel association feature into the target time feature, the target channel association feature can be separately embedded into each intermediate time feature to obtain an initial embedded feature corresponding to each intermediate time feature, and the target embedded feature is formed by the initial embedded features.

In all embodiments of the present disclosure, features may be represented by a matrix. When the target channel association feature is embedded into the target time feature to obtain the target embedded feature, specifically, multiplication processing may be performed on two matrices to obtain a target embedding matrix that represents the target embedded feature.

Step S208. Extract a spatial feature from the obtained embedded feature, and obtain a classification result of the electrophysiological signal based on the spatial feature. In some embodiments of the present disclosure, the spatial feature can be preferably configured as the target spatial feature.

Specifically, after obtaining the target embedded feature, the computer device may further perform spatial feature extraction on the to-be-classified electrophysiological signal to obtain a target spatial feature corresponding to the to-be-classified electrophysiological signal, and then obtain a final classification result based on the target spatial feature.

In all embodiments of the present disclosure, the computer device can perform multiple spatial feature extractions on the target embedded feature to obtain multiple intermediate spatial features, and the target spatial feature is formed by the intermediate spatial features.

In all embodiments of the present disclosure, the computer device can perform classification processing on the to-be-classified electrophysiological signal by means of a machine learning model to obtain a classification result of the to-be-classified electrophysiological signal. The computer device can integrate the channel association feature into a processing layer of the electrophysiological signal classification model in advance, so as to integrate the channel association feature into the electrophysiological signal classification model. In this case, after acquiring the to-be-classified electrophysiological signal collected by the target acquisition device, the computer device can acquire a target electrophysiological signal classification model corresponding to the target acquisition device, input the to-be-classified electrophysiological signal into the target electrophysiological signal classification model, and perform data processing on the to-be-classified electrophysiological signal through the target electrophysiological signal classification model to obtain a corresponding classification result. The target electrophysiological signal classification model is integrated into the target channel association feature corresponding to the target acquisition device.

In all embodiments of the present disclosure, the obtaining a classification result corresponding to the to-be-classified electrophysiological signal based on the target spatial feature can include: performing nonlinear processing on the target spatial feature to obtain a target fitting feature; and performing classification processing on the target fitting feature to obtain the classification result.

Specifically, in the process of obtaining the classification result based on the target spatial feature, the computer device can perform nonlinear processing on the target spatial feature to obtain a target fitting feature, and then perform classification processing on the target fitting feature to obtain the classification result. The nonlinear processing can remove some redundant data in the target spatial feature, introduce a nonlinear factor, and enhance expression capability of a model. The classification processing is used for determining a final classification result.

In all embodiments of the present disclosure, the computer device can perform classification processing on the to-be-classified electrophysiological signal by means of a machine learning model. In this case, the computer device may specifically perform the nonlinear processing through an activation function in a nonlinear layer of the target electrophysiological signal classification model, and perform classification processing through a fully-connected layer in the target electrophysiological signal classification model to output the classification result. In some embodiments, above operation of performing the nonlinear processing through an activation function comprises: inputting the target spatial feature, as the input parameters, to the activation function (for instance, Sigmoid function, or ,Tanh function, or Relu function), and executing the activation function to obtain output feature. In some embodiments, above operation of performing the classification processing through a fully-connected layer comprises: inputting the output feature to the fully-connected layer, and multiplying the output feature by a predetermined weight matrix to obtain the classification result.

In the method for classification processing of an electrophysiological signal, a to-be-classified electrophysiological signal collected by a target acquisition device is acquired; a target channel association feature corresponding to the target acquisition device is acquired, the target channel association feature being generated based on spatial locations of multiple acquisition channels on the target acquisition device; a target time feature corresponding to the to-be-classified electrophysiological signal is extracted, and the target channel association feature is embedded into the target time feature to obtain a target embedded feature; and a target spatial feature corresponding to the target embedded feature is extracted, and a classification result corresponding to the to-be-classified electrophysiological signal is obtained based on the target spatial feature. In this way, the target channel association feature is generated based on the spatial locations of the multiple acquisition channels on the target acquisition device, which can represent a topology feature of the electrophysiological signal. During processing of the electrophysiological signal, time domain and frequency domain features as well as the topology feature of the electrophysiological signal is considered comprehensively, and finally an accurate classification result can be obtained, thereby improving classification accuracy of the electrophysiological signal.

In all embodiments of the present disclosure, the acquiring a channel association feature can include:

acquiring an electrophysiological signal classification model corresponding to the acquisition device, the electrophysiological signal classification model being integrated with the channel association feature, and being configured to extract the time feature from the collected electrophysiological signal, embedding the channel association feature into the time feature to obtain the embedded feature, extracting the spatial feature from the obtained embedded feature, and obtaining the classification result of the electrophysiological signal based on the spatial feature. In some embodiments of the present disclosure, the electrophysiological signal classification model can be preferably configured as the target electrophysiological signal classification model.

The electrophysiological signal classification model is a machine learning model for classifying the electrophysiological signal. There may be multiple electrophysiological signal classification models. Different acquisition devices correspond to different electrophysiological signal classification models, and one acquisition device may correspond to at least one electrophysiological signal classification model. The target electrophysiological signal classification model refers to a target electrophysiological signal classification model that corresponds to the target acquisition device and has been trained.

Specifically, the computer device can integrate the channel association feature into a machine learning model, and perform classification processing on the to-be-classified electrophysiological signal by means of the machine learning model. Therefore, after acquiring the to-be-classified electrophysiological signal collected by the target acquisition device, the computer device can directly acquire the target electrophysiological signal classification model corresponding to the target acquisition device, input the to-be-classified electrophysiological signal into the target electrophysiological signal classification model, extract the target time feature corresponding to the to-be-classified electrophysiological signal through the target electrophysiological signal classification model, embed the target channel association feature into the target time feature to obtain the target embedded feature, extract the target spatial feature corresponding to the target embedded feature through the target electrophysiological signal classification model, and obtain the classification result corresponding to the to-be-classified electrophysiological signal based on the target spatial feature. Finally, the target electrophysiological signal classification model outputs the classification result.

In all embodiments of the present disclosure, the target electrophysiological signal classification model can include a temporal convolution layer, a network embedding layer, a spatial convolution layer, a nonlinear layer and a fully-connected layer. The network embedding layer is integrated with the target channel association feature. The computer device can extract the target time feature corresponding to the to-be-classified electrophysiological signal through the temporal convolution layer, embed the target channel association feature into the target time feature through the network embedding layer to obtain the target embedded feature, extract the target spatial feature corresponding to the target embedded feature through the spatial convolution layer, perform nonlinear processing on the target spatial feature through the nonlinear layer to obtain the target fitting feature, perform classification processing on the target fitting feature through the fully-connected layer to obtain the classification result, and finally output the classification result.

In all embodiments of the present disclosure, locations of acquisition channels can be usually different on different acquisition devices. Therefore, an electrophysiological signal classification model corresponding to each acquisition device needs to be established. One electrophysiological signal classification model is only integrated with the channel association feature corresponding to one acquisition device, and one electrophysiological signal classification model is exclusively used for processing electrophysiological signals collected by a corresponding acquisition device. In addition, one acquisition device may also correspond to multiple electrophysiological signal classification models, and different electrophysiological signal classification models correspond to different classification tasks. For example, in a case that an electrophysiological signal is an EEG signal, a classification task for the EEG signal includes sentiment classification, motion imagination classification, attention classification, or the like, and an EEG signal classification model specially used for sentiment classification, an EEG signal classification model specially used for motion imagination classification and an EEG signal classification model specially used for attention classification can be trained. In a case that an electrophysiological signal is an electromyography (EMG) signal, a classification task for the EMG signal includes sentiment classification, muscle state classification, or the like, and an EMG signal classification model specially used for sentiment classification of the EMG signal and an EMG signal classification model specially used for muscle state classification of the EMG signal can be trained. Therefore, the target electrophysiological signal classification model may be a target electrophysiological signal classification model that corresponds to the target acquisition device and the target classification task.

In all embodiments of the present disclosure, the channel association feature can be integrated into the target electrophysiological signal classification model, and the target channel association feature only needs to be calculated once for different electrophysiological signals collected by the same acquisition device, so that classification efficiency of the electrophysiological signal can be effectively improved. In addition, a series of data processing for the to-be-classified electrophysiological signal can be implemented through the target electrophysiological signal classification model to obtain the classification result, which can further improve the classification efficiency of the electrophysiological signal.

In all embodiments of the present disclosure, the acquiring an electrophysiological signal classification model can include:

determining a classification task; acquiring multiple candidate electrophysiological signal classification models corresponding to the classification task, the multiple candidate electrophysiological signal classification models having corresponding candidate acquisition devices; and determining the electrophysiological signal classification model corresponding to the acquisition device from the candidate electrophysiological signal classification models. In some embodiments of the present disclosure, the classification task can be preferably configured as the target classification task.

Specifically, one acquisition device may correspond to multiple electrophysiological signal classification models. Different electrophysiological signal classification models correspond to different classification tasks. Therefore, the computer device may first determine the target classification task from multiple candidate classification tasks, and acquire multiple candidate electrophysiological signal classification models corresponding to the target classification task. The candidate electrophysiological signal classification models correspond to different candidate acquisition devices. In order to process the to-be-classified electrophysiological signal collected by the target acquisition device, the computer device can determine the target electrophysiological signal classification model corresponding to the target acquisition device from the candidate electrophysiological signal classification models, and then input the to-be-classified electrophysiological signal into the target electrophysiological signal classification model, thereby implementing classification of the to-be-classified electrophysiological signal on the target classification task.

In all embodiments of the present disclosure, the computer device may sequentially use each of the candidate classification tasks as a target classification task. Further, the computer device may finally obtain corresponding classification results of the to-be-classified electrophysiological signal on the classification tasks. For example, in a case that the electrophysiological signal is an EEG signal, a classification task for the electroencephalogram signal includes sentiment classification, motion imagination classification, and attention classification. The computer device may first input the to-be-classified EEG signal into an EEG signal classification model for EEG sentiment classification to obtain a sentiment classification result, then input the to-be-classified EEG signal into an EEG signal classification model for EEG motion imagination classification to obtain a motion imagination classification result, and finally input the to-be-classified EEG signal into an EEG signal classification model for EEG attention classification to obtain an attention classification result.

In all embodiments of the present disclosure, the computer device can acquire an electrophysiological signal classification request, the electrophysiological signal classification request carrying a target task identifier and the to-be-classified electrophysiological signal collected by the target acquisition device. The electrophysiological signal classification request is used for requesting classification of the to-be-classified electrophysiological signal. The computer device can determine the target classification task according to the target task identifier, determine the target electrophysiological signal classification model corresponding to the target acquisition device from multiple candidate electrophysiological signal classification models corresponding to the target classification task, and input the to-be-classified electrophysiological signal into the target electrophysiological signal classification model, thereby obtaining a classification result corresponding to the to-be-classified electrophysiological signal. The electrophysiological signal classification request can be generated according to a trigger operation acted by a user on a related interface of the terminal. For example, the user can select the target classification task and the to-be-classified electrophysiological signal collected by the target acquisition device on the related interface, and then trigger generation of the electrophysiological signal classification request. The electrophysiological signal classification request can also be automatically generated. For example, once the target acquisition device acquires the electrophysiological signal, the terminal automatically generates the electrophysiological signal classification request. The target task identifier in the electrophysiological signal classification request can include a task identifier corresponding to any one of the candidate classification tasks, and can also include task identifiers corresponding to the candidate classification tasks respectively.

In all embodiments of the present disclosure, one acquisition device can correspond to multiple electrophysiological signal classification models, and different electrophysiological signal classification models correspond to different classification tasks. The electrophysiological signal classification models have respective functions and focus on data processing of the corresponding classification tasks. In this way, data processing is performed on the to-be-classified electrophysiological signal through a specific electrophysiological signal classification model, which can further improve the accuracy of the classification result.

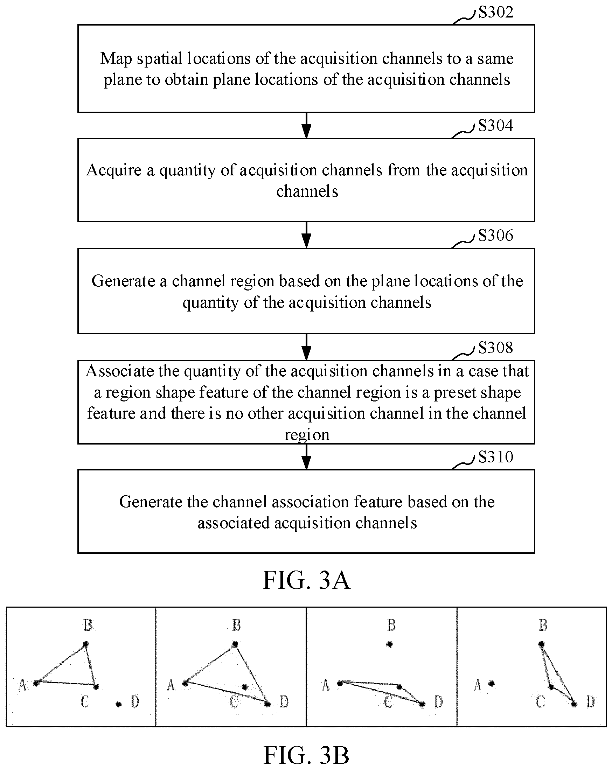

In all embodiments of the present disclosure, as shown in FIG. 3A, the channel association feature can be generated through the following steps:

Step S302. Map spatial locations of the acquisition channels to a same plane to obtain plane locations of the acquisition channels.

Step S304. Acquire a quantity of acquisition channels from the acquisition channels. In some embodiments of the present disclosure, the quantity of acquisition channels can be preferably configured as the target quantity of acquisition channels, and the target quantity of acquisition channels can be preferably as the target acquisition channels.

Step S306. Generate a channel region based on the plane locations of the acquisition channels. In some embodiments of the present disclosure, the channel region can be preferably configured as the target channel region.

Specifically, the computer device can map spatial locations of the acquisition channels to the same plane to obtain plane locations of the acquisition channels in the plane. For example, spatial coordinates of the acquisition channels on the target acquisition device are in the same spatial coordinate system, and the acquisition channels can be projected to xy plane uniformly, so as to obtain plane coordinates of the acquisition channels. Further, the computer device can acquire a target quantity of acquisition channels from the acquisition channels as target acquisition channels to obtain a target quantity of target acquisition channels, and then generate a target channel region based on the plane locations of the target acquisition channels. The target channel region is a plane region surrounded by the target acquisition channels. The computer device may specifically use each of the target acquisition channels as a region vertex to obtain multiple region vertexes, and connect the region vertexes in turn according to a clockwise direction or counterclockwise direction. The target channel region is a plane region surrounded by the region vertexes and vertex connection lines. The target quantity may be set according to actual needs. For example, in a case that the target quantity is three, the computer device may determine all acquisition channel combinations that include three acquisition channels in the acquisition channels to obtain multiple acquisition channel combinations. The acquisition channel combinations each include three target acquisition channels, and each of the acquisition channel combinations can generate one target channel region. In one acquisition channel combination, the computer device may specifically use each of the three acquisition channels as a region vertex to obtain three region vertexes, and connect the region vertexes in turn according to a clockwise direction or counterclockwise direction. The target channel region is a triangular plane region surrounded by the region vertexes and connection lines.

Step S308. Associate the quantity of the acquisition channels in a case that a region shape feature of the channel region is a preset shape feature and there is no other acquisition channel in the channel region.

The region shape feature is feature information for describing a region shape of the target channel region.

Specifically, in a case that the region shape feature corresponding to the target channel region is a preset shape feature and there is no other acquisition channel in the target channel region, it indicates that the acquisition channels included in the target channel region are closely linked to each other. In this case, the acquisition channels corresponding to the target channel region can be associated. Therefore, in a case that two acquisition channels are associated, it can be determined that the two acquisition channels are closely linked to each other.

In all embodiments of the present disclosure, vertex angles corresponding to region vertexes of a region may be used for reflecting a region shape feature of the region. For example, whether a triangle is an acute triangle, a right triangle, or an obtuse triangle can be determined according to the vertex angles corresponding to the three vertexes of the triangle. Whether a quadrangle is a rectangular, a trapezoid, or a rhombus can be determined according to the vertex angles corresponding to the four vertexes of the quadrangle. Therefore, the computer device can calculate vertex angles corresponding to the region vertexes of the target channel region and obtain the region shape feature corresponding to the target channel region based on the vertex angles. Correspondingly, the preset shape feature can be represented by using a reference angle range of the vertex angles. In a case that the vertex angles corresponding to the target channel region are all within the reference angle range, it can be determined that the region shape feature corresponding to the target channel region is the preset shape feature. For example, in a case that the target channel region is a triangular region, the preset shape feature may be that the vertex angles are all within a range from 0 to 90 degrees, that is, in a case that the target channel region is an acute triangle region or a right triangle region, it is determined that the region shape feature corresponding to the target channel region is the preset shape feature.

In all embodiments of the present disclosure, alternatively, a preset channel region having the preset shape feature can be obtained, a similarity between the target channel region and the preset channel region is calculated, and it is determined that a region shape feature corresponding to the target channel region with a similarity greater than a preset threshold is the preset shape feature.

Step S310. Generate the channel association feature based on the associated acquisition channels.

Specifically, after determining associations between the acquisition channels, the computer device can generate the target channel association feature based on the associated acquisition channels. The computer device can generate an adjacency matrix corresponding to the target acquisition device based on the associated acquisition channels and obtain the target channel association feature based on the adjacency matrix. Matrix dimensionality of the adjacency matrix is a channel quantity of the acquisition channels on the target acquisition device, and matrix values in the adjacency matrix can reflect whether any two acquisition channels are associated. For example, in a case that the matrix value is 0, it indicates that the corresponding acquisition channels are not associated; in a case that the matrix value is 1, it indicates that the corresponding acquisition channels are associated.

In all embodiments of the present disclosure, spatial locations of the acquisition channels can be mapped to the same plane to obtain plane locations of the acquisition channels; a target quantity of acquisition channels are acquired from the acquisition channels as target acquisition channels to obtain the target quantity of target acquisition channels;

a target channel region is generated based on the plane locations of the target acquisition channels; and the target acquisition channels are associated in a case that a region shape feature corresponding to the target channel region is a preset shape feature and there is no other acquisition channel in the target channel region; and the target channel association feature is generated based on the associated acquisition channels. In this way, closely linked acquisition channels can be quickly found by analyzing the plane locations of the target acquisition channels, thereby generating an accurate target channel association feature.

In all embodiments of the present disclosure, the generating a target channel region based on the plane locations of the target acquisition channels can include:

using each of the target acquisition channels as a region vertex to obtain multiple region vertexes, and connecting the region vertexes in turn to obtain the target channel region.

Specifically, when generating the target channel region, the computer device may use each of the target acquisition channels as a region vertex to obtain multiple region vertexes, and connect the region vertexes in turn according to a clockwise direction or counterclockwise direction. A plane region surrounded by the vertex connection lines is the target channel region corresponding to the current acquisition channels. In this way, the target channel region can be generated quickly.

In all embodiments of the present disclosure, the region shape feature can include vertex angles corresponding to the region vertexes of the channel region, and the associating the quantity of the acquisition channels in a case that a region shape feature of the channel region is a preset shape feature and there is no other acquisition channel in the channel region can include:

determining that the region shape feature of the channel region is the preset shape feature in a case that the vertex angles are all within a reference angle range; and associating the quantity of the acquisition channels based on connection relationships between the region vertexes in a case that there is no other acquisition channel in the channel region of which the region shape feature is the preset shape feature.

Specifically, vertex angles corresponding to region vertexes of a region may be used for reflecting a region shape feature of the region. Therefore, the computer device can calculate vertex angles corresponding to the region vertexes of the target channel region, and the region shape feature corresponding to the target channel region is formed by the vertex angles corresponding to the region vertexes of the target channel region. That is, the region shape feature includes the vertex angles corresponding to the region vertexes of the target channel region. The computer device may specifically calculate the vertex angles corresponding to the region vertexes according to plane distances between the region vertexes.

Correspondingly, in a case that a shape feature of a region is a preset shape feature, vertex angles corresponding to the region have a certain characteristic. Therefore, a reference angular range corresponding to vertex angles of a channel region whose region shape feature is the preset shape feature may be preset. In a case that each vertex angle is within the reference angle range, the computer device may determine that the region shape feature corresponding to the target channel region is the preset shape feature. Further, in a case that there is no other acquisition channel in the target channel region whose region shape feature is the preset shape feature, it indicates that there is no other redundant acquisition channel in the target channel region. The target acquisition channels forming the target channel region are a combination of closely linked acquisition channels. In this case, the target acquisition channels can be associated based on the associations between the region vertexes.

In all embodiments of the present disclosure, the computer device may set the target quantity to three because the triangle is the most stable and dense polygon. Further, in a case that there is no other node except the three vertexes in the triangle, and the triangle is an acute triangle or a right triangle, the three vertexes of the triangle may be considered as three most closely associated nodes. In a case that there is no other node except the three vertexes in the triangle, it indicates that the three vertexes are three nodes with relatively close plane distances and no other interference inside. Further, in a case that the triangle is an acute triangle or a right triangle, it indicates that the distances between the three vertexes are appropriate and are not significantly different. In a case that the triangle is an obtuse triangle, the side corresponding to the obtuse angle is obviously much longer than the other two sides, that is, two vertexes in the obtuse triangle are relatively far apart, and the connection between the two vertexes is less tight. In a case that the three vertex angles of the generated triangle are all within a range of 0 to 90 degrees, it can be determined that the triangle is an acute triangle or a right triangle rather than an obtuse triangle. Therefore, in a case that there is no other node except the three vertexes in the triangle, and the triangle is an acute triangle or a right triangle, the target acquisition channels may be associated according to side lengths of the triangle. The two target acquisition channels connected by each side are associated. In this way, three sets of associated target acquisition channels can be obtained from one triangle.

For example, assuming that there are four acquisition channels: A, B, C, and D; three acquisition channels are arbitrarily selected from the four acquisition channels as target acquisition channels, and the three target acquisition channels are used as triangular vertexes. The triangular vertexes are connected in turn to obtain a triangle. A region surrounded by the triangle is the target channel region. As shown in FIG. 3B, a total of four triangles can be established: A-B-C, A-B-D, A-C-D, and B-C-D. The triangle A-B-C is an acute triangle without D inside. Therefore, A and B can be associated, B and C can be associated, and A and C can be associated. Although the triangle A-B-D an acute triangle, C exists inside the triangle. Therefore, there is no need to associate A, B and D. The triangle A-C-D triangle is an obtuse triangle. Therefore, there is no need to associate A, C and D. The triangle B-C-D triangle is an obtuse triangle. Therefore, there is no need to associate B, C and D.

In all embodiments of the present disclosure, in addition to a triangle, the target channel region may be a quadrangle, that is, the computer device may also set the target quantity to four. In a case that the target channel region is a quadrangular region, the target channel region can be divided into two triangles, and there are two division manners. In a case that neither of the triangles is an obtuse triangle and there is no other acquisition channel in the triangular regions, corresponding acquisition channels can be associated according to connecting edges of the two triangles.

In all embodiments of the present disclosure, whether the region shape feature corresponding to the target channel region is a preset shape feature can be quickly determined based on the vertex angles corresponding to the region vertexes of the target channel region.

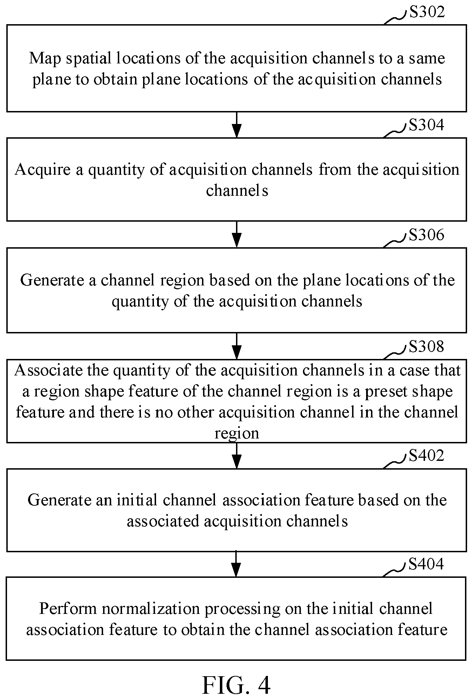

In all embodiments of the present disclosure, as shown in FIG. 4, the generating the channel association feature based on the associated acquisition channels can include:

Step S402. Generate an initial channel association feature based on the associated acquisition channels.
Step S404. Perform normalization processing on the initial channel association feature to obtain the channel association feature.

The normalization processing is used for limiting the initial channel association feature within a certain interval, so that the target channel association feature can be embedded into the target time feature subsequently. The normalization processing can be performed on the initial channel association feature through a custom formula to obtain the target channel association feature.

Specifically, in the process of generating the target channel association feature based on the associated acquisition channels, the computer device may directly generate the target channel association feature based on the associated acquisition channels. However, in order to subsequently embed the target channel association feature into the target time feature without changing an assignment range of an original signal, the computer device can also first generate the initial channel association feature based on the associated acquisition channels, and obtain the target channel association feature after performing normalization processing on the initial channel association feature.

In all embodiments of the present disclosure, the channel association feature may be represented by a matrix, matrix dimensionality of the matrix is a channel quantity of the acquisition channels on the target acquisition device. Values in rows and columns of the target channel association matrix obtained by the normalization processing are all 1. Subsequently, in a case that the target channel association feature is embedded into the target time feature, matrix multiplication can be performed. If values in the rows and columns of the target channel association matrix are all 1, the matrix multiplication does not change the assignment range of the original signal, that is, the original signal does not increase or decrease sharply, thereby reserving the assignment interval of the original signal to a certain extent.

In all embodiments of the present disclosure, the initial channel association feature can be generated based on the associated acquisition channels, and normalization processing can be performed on the initial channel association feature to obtain the target channel association feature. The normalization processing can improve validity and accuracy of the target channel association feature, so that the target channel association feature is conveniently embedded into the target time feature subsequently.

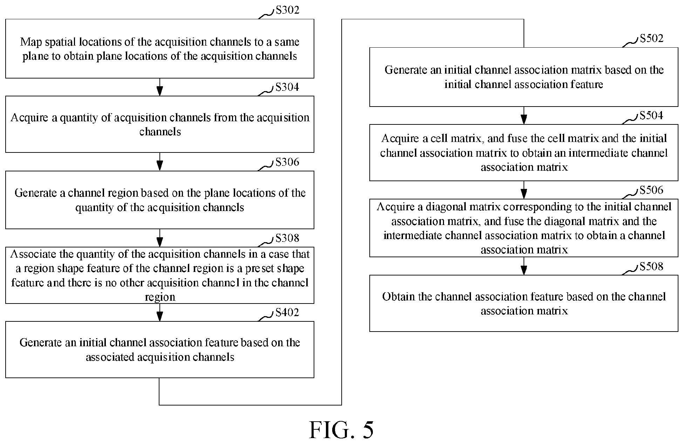

In all embodiments of the present disclosure, as shown in FIG. 5, the performing normalization processing on the initial channel association feature to obtain the channel association feature can include:
Step S502. Generate an initial channel association matrix based on the initial channel association feature.

Specifically, the computer device can generate the initial channel association matrix based on the initial channel association feature. The initial channel association feature is expressed in a matrix form to facilitate subsequent calculation processing.

In an embodiment, the generating an initial channel association matrix based on the initial channel association feature includes any one of the following manners:

determining matrix dimensionality of the initial channel association matrix based on the channel quantity of the acquisition channels on the target acquisition device, setting matrix values corresponding to the associated acquisition channels to a first preset threshold, and setting matrix values corresponding to other acquisition channels to a second preset threshold, to obtain the initial channel association matrix; or

determining matrix dimensionality of the initial channel association matrix based on the channel quantity of the acquisition channels on the target acquisition device, determining matrix values corresponding to the associated acquisition channels based on spatial location distances between the associated acquisition channels, and setting matrix values corresponding to other acquisition channels to a second preset threshold, to obtain the initial channel association matrix.

Specifically, the initial channel association matrix may be an unweighted network, which only includes associations between the acquisition channels. Therefore, in the process of generating the initial channel association matrix, the computer device can use the channel quantity of the acquisition channels on the target acquisition device as matrix dimensionality of the initial channel association matrix, set matrix values corresponding to the associated acquisition channels to a first preset threshold, and set matrix values corresponding to other acquisition channels to a second preset threshold, to obtain the initial channel association matrix. The first preset threshold and the second preset threshold may be set as required.

For example, the acquisition channels on the target acquisition device include A, B, C and D, and the channel quantity is 4. A, B and D can form an acute triangle without C inside, and B, C and D can form an acute triangle without A inside. Therefore, A-B, A-D, B-D, B-C and C-D have associations respectively. If the first preset threshold is set to 1 and the second preset threshold is set to 0, the generated initial channel association matrix can be:

$$\begin{matrix} 0 & 1 & 0 & 1 \\ 1 & 0 & 1 & 1 \\ 0 & 1 & 0 & 1 \\ 1 & 1 & 1 & 0 \end{matrix}$$

Row elements of the initial channel association matrix represent A, B, C and D in turn, and column elements represent A, B, C and D in turn.

The initial channel association matrix alternatively may be a weighted network, which includes associations and spatial location distances between the acquisition channels. Therefore, in the process of generating the initial channel association matrix, the computer device can determine matrix dimensionality of the initial channel association matrix based on the channel quantity of the acquisition channels on the target acquisition device, determine matrix values corresponding to the associated acquisition channels based on spatial location distances between the associated acquisition channels, and set matrix values corresponding to other acquisition channels to the second preset threshold, to obtain the initial channel association matrix. A reciprocal of the spatial location distance may be used as a matrix value.

For example, the acquisition channels on the target acquisition device include A, B, C and D, and the channel quantity is 4. A-B, A-D, B-D, B-C and C-D have associations respectively. The spatial location distance between A and B is x1, the spatial location distance between A and D is x2, the spatial location distance between B and D is x3, the spatial location distance between B and C is x4, and the spatial location distance between C and D is x5. The reciprocal of the spatial location distance can be used as the matrix value.

In this case, the generated initial channel association matrix can be:

$$\begin{matrix} 0 & \frac{1}{x1} & 0 & \frac{1}{x2} \\ \frac{1}{x1} & 0 & \frac{1}{x4} & \frac{1}{x3} \\ 0 & \frac{1}{x4} & 0 & \frac{1}{x5} \\ \frac{1}{x2} & \frac{1}{x3} & \frac{1}{x5} & 0 \end{matrix}$$

Row elements of the initial channel association matrix represent A, B, C and D in turn, and column elements represent A, B, C and D in turn.

Step S504. Acquire a cell matrix, and fuse the cell matrix and the initial channel association matrix to obtain an intermediate channel association matrix.

Step S506. Acquire a diagonal matrix corresponding to the initial channel association matrix, and fuse the diagonal matrix and the intermediate channel association matrix to obtain a channel association matrix. In some embodiments of the present disclosure, the channel association matrix can be preferably configured as the target channel association matrix.

Step S508. Obtain the channel association feature based on the channel association matrix.

The cell matrix refers to a square matrix in which diagonal elements are non-zero and other elements are zero. The diagonal matrix refers to a matrix in which all elements except a main diagonal are zero. The diagonal matrix corresponding to the initial channel association matrix refers to a matrix in which elements on a main diagonal are determined according to matrix values of the initial channel association matrix, while elements except the main diagonal are all zero.

Specifically, in order to subsequently embed the target channel association feature into the target time feature without losing respective electrophysiological signals of the acquisition channels, the computer device can acquire the cell matrix, fuse the cell matrix and the initial channel association matrix to obtain an intermediate channel association matrix. For example, the cell matrix is a square matrix in which elements on the diagonal are 1 and other elements are 0. The matrix dimensionality of the cell matrix is the channel quantity of the acquisition channels on the target acquisition device. The computer device can perform matrix addition processing on the cell matrix and the initial channel association matrix to obtain an intermediate channel association matrix. Because the elements on the diagonal of the initial channel association matrix are 0, if the initial channel association matrix is not fused with the cell matrix, the target embedded feature obtained by subsequent calculation does not include related information of the respective electrophysiological signals of the acquisition channels, which is not conducive to the classification of electrophysiological signals.

Further, in order to subsequently embed the target channel association feature into the target time feature without changing the assignment interval of the original signal, values in rows and columns of the intermediate channel association matrix need to be converted into 1. Therefore, the computer device can acquire the diagonal matrix corresponding to the initial channel association matrix, fuse the diagonal matrix and the intermediate channel association matrix to obtain a target channel association matrix. Finally, the computer device can obtain the target channel association feature based on the target channel association matrix. For example, the target channel association matrix is used as the target channel association feature.

In all embodiments of the present disclosure, the normalization processing can be implemented by the following formula:

$$\hat{A} = D^{-\frac{1}{2}} \tilde{A} D^{-\frac{1}{2}}, \quad \tilde{A} = A + I, \quad D = diag\left(\sum_j \widetilde{A_{1,j}}, \sum_j \widetilde{A_{2,j}}, \ldots \ldots, \sum_j \widetilde{A_{n,j}}\right)$$

A represents the initial channel association matrix, which may also be referred to as an initial adjacency matrix. I represents the cell matrix. $\tilde{A}$ represents the intermediate channel association matrix. D represents the diagonal matrix corresponding to the initial channel association matrix, and $diag\left(\sum_j \widetilde{A_{1,j}}, \sum_j \widetilde{A_{2,j}}, \ldots \ldots, \sum_j \widetilde{A_{n,j}}\right)$ represents elements on the diagonal line of the diagonal matrix. $\sum_j \widetilde{A_{1,j}}$ represents a sum of all elements in the first row in $\tilde{A}$. $\sum_j \widetilde{A_{2,j}}$ represents a sum of all elements in the second row in $\tilde{A}$. $\sum_j \widetilde{A_{n,j}}$ represents a sum of all elements in the $n^{th}$ row in $\tilde{A}$. n represents the channel quantity of the acquisition channels on the target acquisition device. $\hat{A}$ represents the target channel association matrix.

In all embodiments of the present disclosure, the to-be-classified electrophysiological signal can include electrophysiological signals corresponding to the acquisition channels respectively, and the extracting a time feature from the collected electrophysiological signal includes:

acquiring at least one temporal convolution kernel; separately extracting time sub-features corresponding to the electrophysiological signals based on a same temporal convolution kernel to obtain multiple time sub-features respectively corresponding to each of the at least one temporal convolution kernel; generating an intermediate time feature based on the multiple time sub-features corresponding to the same temporal convolution kernel, to obtain at least one intermediate time feature corresponding to the at least one temporal convolution kernel respectively; and obtaining the time feature based on the at least one intermediate time feature.

The to-be-classified electrophysiological signal is a multi-channel time sequence signal, and the to-be-classified electrophysiological signal includes the electrophysiological signals corresponding to the acquisition channels respectively. The electrophysiological signal can reflect changes in voltage or current over time.

Specifically, different temporal convolution kernels are used for extracting time features with different viewing angles. For example, one temporal convolution kernel focuses on extracting a starting time feature of the signal, and one temporal convolution kernel focuses on extracting an end time feature of the signal. In the process of extracting the target time feature corresponding to the to-be-classified electrophysiological signal, the computer device can separately extract time features of the electrophysiological signals corresponding to the acquisition channels based on the temporal convolution kernel, that is, separately extract the time sub-features corresponding to the electrophysiological signals based on the same temporal convolution kernel. Because there are multiple temporal convolution kernels, multiple time sub-features corresponding to each temporal convolution kernel can be finally be obtained. Further, the computer device may generate an intermediate time feature based on the multiple time sub-features corresponding to the same temporal convolution kernel. For example, the multiple time sub-features corresponding to the same temporal convolution kernel is spliced to obtain the intermediate time feature. Finally, the computer device may obtain the target time feature based on the intermediate time features. For example, the target time feature is formed by the intermediate time features.

In all embodiments of the present disclosure, the size of the to-be-classified electrophysiological signal can be T*C, where T represents a length of a single electrophysiological signal, that is, a time range corresponding to the electrophysiological signal, and C represents a channel quantity. The temporal convolution kernel is a two-dimensional convolution kernel with a size of n* 1, where the value of n is adjustable. Different temporal convolution kernels may correspond to the same or different n, and matrix values of different temporal convolution kernels can be the same or different. The temporal convolution kernel only performs convolution on the to-be-classified electrophysiological signal in the time dimension, and only extracts the time feature.

In all embodiments of the present disclosure, the target electrophysiological signal classification model can include a temporal convolution layer for extracting a time feature of the to-be-classified electrophysiological signal. The temporal convolution layer includes at least one temporal convolution kernel. After inputting the to-be-classified electrophysiological signal into the target electrophysiological signal classification model, the computer device can extract the target time feature corresponding to the to-be-classified electrophysiological signal through the temporal convolution layer. In the temporal convolution layer, time sub-features corresponding to the electrophysiological signals are extracted based on a same temporal convolution kernel to obtain multiple time sub-features corresponding to each of the temporal convolution kernels; an intermediate time feature is generated based on the multiple time sub-features corresponding to the same temporal convolution kernel, to obtain intermediate time features corresponding to the temporal convolution kernels; and the target time feature based on the intermediate time features.

In all embodiments of the present disclosure, the temporal convolution layer can include at least one temporal convolution kernel, and different temporal convolution kernels can be used for extracting time features with different viewing angles, so that the target time feature can include time features with multiple angles, thereby improving richness and accuracy of the target time feature.

In all embodiments of the present disclosure, the time feature can include multiple intermediate time features, and the embedding the channel association feature into the time feature to obtain an embedded feature includes: separately embedding the channel association feature into the intermediate time features to obtain initial embedded features corresponding to the intermediate time features, and obtaining the embedded feature based on the initial embedded features.

Specifically, in a case that the target time feature is a single time feature matrix, the computer device can directly embed the target channel association feature into the target time feature to obtain the target embedded feature. In a case that the target time feature includes multiple time feature matrices, that is, the target time feature includes multiple intermediate time features, the computer device needs to separately embed the target channel association feature into the intermediate time features to obtain initial embedded features corresponding to the intermediate time features, and then the target embedded feature is generated from the initial embedded features.

In all embodiments of the present disclosure, in a case that the target time feature includes multiple intermediate time features, the target channel association feature needs to be separately embedded into the intermediate time features to obtain the initial embedded features corresponding to the intermediate time features, and the target embedded feature is obtained based on the initial embedded features. In this way, a more accurate target embedded feature can be obtained.

In all embodiments of the present disclosure, the channel association feature can include association sub-features corresponding to the acquisition channels respectively, the intermediate time feature can include time sub-features corresponding to the acquisition channels respectively. The separately embedding the channel association feature into the intermediate time features to obtain initial embedded features corresponding to the intermediate time features includes:
in a current intermediate time feature, embedding an association sub-feature corresponding to a same acquisition channel into a corresponding time sub-feature, to obtain embedded sub-features corresponding to the acquisition channels respectively; and obtaining an initial embedded feature corresponding to the current intermediate time feature based on the embedded sub-features.

Specifically, the channel association feature includes association sub-features corresponding to the acquisition channels respectively, and the intermediate time feature includes time sub-features corresponding to the acquisition channels respectively. In a case that the target channel association feature is embedded into the intermediate time feature, the association sub-feature and time sub-feature corresponding to the same acquisition channel need to be embedded. Therefore, using the current intermediate time feature as an example, the computer device can embed the association sub-feature corresponding to the same acquisition channel into the corresponding time sub-feature, to obtain embedded sub-features corresponding to the acquisition channels respectively; and then an initial embedded feature corresponding to the current intermediate time feature is generated from the embedded sub-features. Similarly, initial embedded features corresponding to the intermediate time features respectively can be finally obtained by performing similar processing on other intermediate time features.

In all embodiments of the present disclosure, the association sub-feature and time sub-feature corresponding to the same acquisition channel can be embedded to obtain a more accurate initial embedded feature.

In all embodiments of the present disclosure, the extracting a spatial feature from the obtained embedded feature can include:

acquiring at least one spatial convolution kernel; separately performing spatial feature extraction on the embedded feature based on the at least one spatial convolution kernel to obtain at least one intermediate spatial feature corresponding to the at least one spatial convolution kernel respectively; and obtaining the spatial feature based on the at least one intermediate spatial feature.

Specifically, different spatial convolution kernels are used for extracting spatial features with different viewing angles. In the process of extracting the target spatial feature corresponding to the target embedded feature, the computer device can perform spatial feature extraction on the target embedded feature based on the spatial convolution kernel to obtain an intermediate spatial feature. Because there are multiple spatial convolution kernels, respective intermediate spatial features corresponding to the spatial convolution kernels can be obtained finally. Further, the computer device can obtain the target spatial feature based on the intermediate spatial features. For example, the target spatial feature is formed by the intermediate spatial features.

In all embodiments of the present disclosure, the size of the to-be-classified electrophysiological signal can be T*C, where T represents the length of a single electrophysiological signal, that is, a time range corresponding to the electrophysiological signal, and C represents a channel quantity. The spatial convolution kernel is a two-dimensional convolution kernel with a size of 1*C. Different spatial convolution kernels have different matrix values. The spatial convolution kernel only performs convolution on the target embedded feature in a channel dimension, fuses embedded features corresponding to different acquisition channels, and only extracts a spatial feature.

In all embodiments of the present disclosure, the target electrophysiological signal classification model can include a spatial convolution layer for extracting the spatial feature of the to-be-classified electrophysiological signal, and the spatial convolution layer can include at least one spatial convolution kernel. The computer device can extract the target spatial feature corresponding to the target embedded feature through the target electrophysiological signal classification model. In the spatial convolution layer, spatial feature extraction is performed on the target embedded feature separately based on the at least one spatial convolution kernel to obtain at least one intermediate spatial features corresponding to the at least one spatial convolution kernel respectively; and the target spatial feature is obtained based on the at least one intermediate spatial feature.

In all embodiments of the present disclosure, the spatial convolution layer can include at least one spatial convolution kernel, and different spatial convolution kernels can be used for extracting spatial features with different viewing angles, so that the target spatial feature may include spatial features with multiple angles, thereby improving richness and accuracy of the target spatial feature.

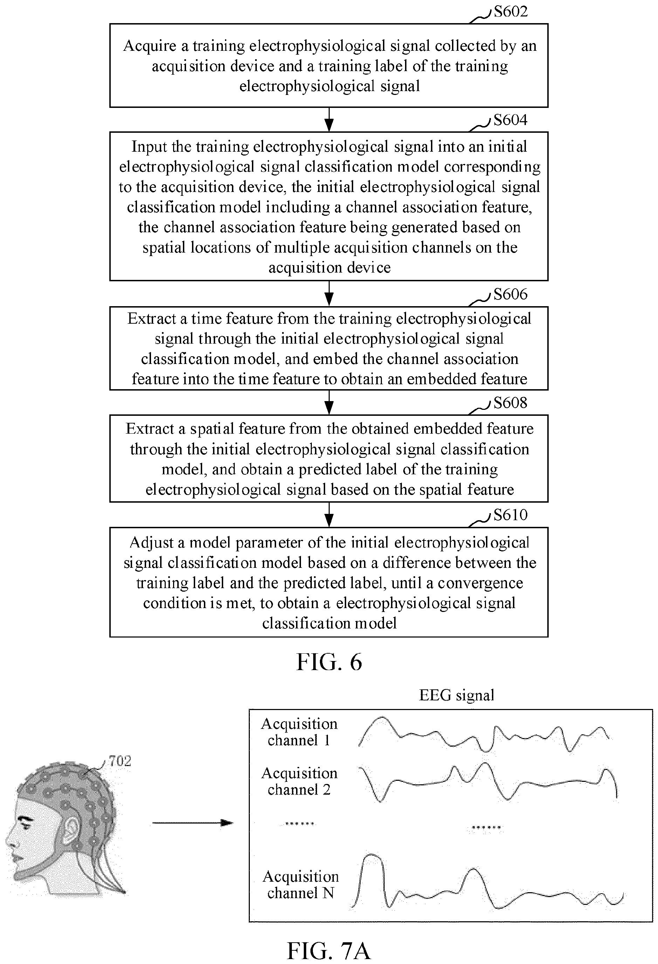

In all embodiments of the present disclosure, as shown in FIG. 6, an exemplary method for classification processing of an electrophysiological signal is provided, which is described by using an example that the method is applied to a computer device shown in FIG. 1. The computer device may be the terminal 102 or the server 104 in FIG. 1. Referring to FIG. 6, the method for classification processing of an electrophysiological signal includes the following steps:

Step S602. Acquire a training electrophysiological signal collected by an acquisition device and a training label of the training electrophysiological signal. In some embodiments of the present disclosure, the acquisition device can be preferably configured as the target acquisition device.

The training electrophysiological signal refers to an electrophysiological signal used for model training. The training label refers to a correct classification result corresponding to the training electrophysiological signal.

Specifically, the computer device can acquire the training electrophysiological signal collected by the target acquisition device and the training label corresponding to the training electrophysiological signal, and perform supervised training on an initial electrophysiological signal classification model corresponding to the target acquisition device based on the training electrophysiological signal and the corresponding training label to obtain a target electrophysiological signal classification model corresponding to the target acquisition device. For example, the computer device can train an EEG signal classification model based on an EEG signal with a known classification result, train an EMG signal with a known classification result, and train an ECG signal with a known classification result.

Step S604. Input the training electrophysiological signal into an initial electrophysiological signal classification model corresponding to the acquisition device, the initial electrophysiological signal classification model including a channel association feature, the channel association feature being generated based on spatial locations of multiple acquisition channels on the acquisition device. In some embodiments of the present disclosure, the channel association feature can be preferably configured as the target channel association feature.

The initial electrophysiological signal classification model refers to an electrophysiological signal classification model to be trained.

Specifically, the computer device can generate a target channel association feature corresponding to the target acquisition device based on the spatial locations of the multiple acquisition channels on the target acquisition device, and integrate the target channel association feature into the initial electrophysiological signal classification model corresponding to the target acquisition device. Further, the computer device can acquire the training electrophysiological signal collected by the target acquisition device and the training label corresponding to the training electrophysiological signal, and perform supervised training on the initial electrophysiological signal classification model based on the training electrophysiological signal and the corresponding training label to obtain the target electrophysiological signal classification model.

For the specific process of generating the target channel association feature, reference may be made to the content of the foregoing related embodiments, and details are not described herein.

Step S606. Extract a time feature from the training electrophysiological signal through the initial electrophysiological signal classification model, and embed the channel association feature into the time feature to obtain an embedded feature. In some embodiments of the present disclosure, the time feature can be preferably configured as the target time feature, and the embedded feature can be preferably configured as the target embedded feature.

Step S608. Extract a spatial feature from the obtained embedded feature through the initial electrophysiological signal classification model, and obtain a predicted label of the training electrophysiological signal based on the spatial feature. In some embodiments of the present disclosure, the spatial feature can be preferably configured as the target spatial feature.

The predicted label refers to a predicted classification result outputted by the initial electrophysiological signal classification model.

Specifically, the computer device can extract the target time feature corresponding to the training electrophysiological signal through the initial electrophysiological signal classification model, embed the target channel association feature into the target time feature to obtain the target embedded feature, extract the target spatial feature corresponding to the target embedded feature, and obtain the predicted label corresponding to the training electrophysiological signal based on the target spatial feature.

For the specific process of performing data processing on the inputted data by the model, reference may be made to the content of the foregoing related embodiments, and details are not described herein.

In all embodiments of the present disclosure, the initial electrophysiological signal classification model can be a lightweight convolutional neural network. A lightweight network is more suitable for a training set with a small quantity of samples, and can effectively suppress overfitting. A training sample of the initial electrophysiological signal classification model (that is, a training electrophysiological signal $X \in R^{N+T+C}$) is used as an input of the network, where N represents a sample quantity, T represents a single-channel length of a sample, that is, a time range corresponding to a single-channel electrophysiological signal, and C represents a channel quantity of the sample. The initial electrophysiological signal classification model includes a temporal convolution layer, a network embedding layer, a spatial convolution layer, a nonlinear layer and a fully-connected layer. First, the training sample is transformed, to expand an original three-dimensional sample to be four-dimensional, where a dimension with a size of 1 is added to the second dimension. That is, the size of the original sample is N*T*C, and the size of the sample after transformation is N* 1 *T*C. The transformed sample is inputted to the temporal convolution layer, and a time feature corresponding to the electrophysiological signal of each acquisition channel is extracted through the temporal convolution layer to obtain a time feature map (that is, the target time feature) $X^t \in R^{N+D+T+C}$. D represents a feature map depth, that is, a feature map quantity, and one temporal convolution kernel corresponds to one feature map. The temporal convolution layer only performs convolution in the time dimension and only extracts a time feature. Then, a time feature map is multiplied by a normalized network adjacency matrix (that is, the target channel association feature) through the network embedding layer, and network embedding is performed to obtain a feature map after the network embedding $X^g \in R^{N*D*T*C}$. Then, the feature map after the network embedding is inputted to the spatial convolution layer for spatial fusion, and nonlinear fitting capability of the network is enhanced through the nonlinear layer. Finally, one fully-connected layer is used for performing feature classification to obtain the predicted label. The spatial convolution layer only performs convolution in the channel dimension.

Step S610. Adjust a model parameter of the initial electrophysiological signal classification model based on a difference between the training label and the predicted label, until a convergence condition is met, to obtain a electrophysiological signal classification model. In some embodiments of the present disclosure, the electrophysiological signal classification model can be preferably configured as the target electrophysiological signal classification model.

The target electrophysiological signal classification model refers to a trained electrophysiological signal classification model.

Specifically, the computer device inputs the training electrophysiological signal into the initial electrophysiological signal classification model corresponding to the target acquisition device to obtain the predicted label corresponding to the training electrophysiological signal, then calculates a training loss value based on the difference between the training label and the predicted label, performs back propagation updating based on the training loss value, and adjusts the model parameter of the initial electrophysiological signal classification model, until the convergence condition is met, to obtain the target electrophysiological signal classification model. The convergence condition may be at least one of the following: the training loss value is less than a loss value threshold, the number of model iterations reaches an iteration threshold, or the like.

In all embodiments of the present disclosure, a cross-entropy loss function can be selected as a loss function of the model. Model parameter updating is performed by using a classical gradient back propagation algorithm, and an Adam optimizer with a default parameter setting can be selected for parameter optimization.

In all embodiments of the present disclosure, one acquisition device may correspond to multiple electrophysiological signal classification models. Different electrophysiological signal classification models correspond to different classification tasks. Therefore, the training label may be a training label corresponding to the training electrophysiological signal under a target classification task. In this case, the computer device obtain the target electrophysiological signal classification model based on the training electrophysiological signal and the corresponding training label. The target electrophysiological signal classification model is designed to output the classification result corresponding to the to-be-classified electrophysiological signal under the target classification task.

In the foregoing method and apparatus for classification processing of an electrophysiological signal, the computer device, and the storage medium, a training electrophysiological signal collected by a target acquisition device and a training label corresponding to the training electrophysiological signal are acquired; the training electrophysiological signal is inputted into an initial electrophysiological signal classification model corresponding to the target acquisition device, the initial electrophysiological signal classification model including a target channel association feature corresponding to the target acquisition device, the target channel association feature being generated based on spatial locations of multiple acquisition channels on the target acquisition device; a target time feature corresponding to the training electrophysiological signal is generated through the initial electrophysiological signal classification model; the target channel association feature is embedded into the target time feature to obtain a target embedded feature; a target spatial feature corresponding to the target embedded feature is extracted through the initial electrophysiological signal classification model; a predicted label corresponding to the training electrophysiological signal is obtained based on the target spatial feature; and a model parameter of the initial electrophysiological signal classification model is adjusted based on a difference between the training label and the predicted label, until a convergence condition is met, to obtain a target electrophysiological signal classification model. In this way, the target channel association feature is generated based on the spatial locations of multiple acquisition channels on the target acquisition device, which can represent a topology feature of the electrophysiological signal. During processing of the electrophysiological signal, time domain and frequency domain features as well as the topology feature of the electrophysiological signal is considered comprehensively, and finally an accurate classification result can be obtained, thereby improving classification accuracy of the electrophysiological signal. In addition, the target channel association feature is integrated into the model, and the target channel association feature only needs to be calculated once for different electrophysiological signals collected by the same acquisition device, thereby effectively improving classification efficiency of the electrophysiological signal.

This application further provides an application scenario, and the above method for classification processing of an electrophysiological signal is applied to the application scenario. Specifically, the application of the method for classification processing of an electrophysiological signal in the application scenario is as follows:

The method for classification processing of an electrophysiological signal provided in this application is applicable to an EEG signal classification task. An EEG signal is an electrophysiological signal obtained by amplifying and recording a scalp electrical signal by an electronic instrument (that is, the acquisition device), and is a multi-channel time sequence. Referring to FIG. 7A, the acquisition device includes multiple electrodes, one electrode corresponds to one acquisition channel, and a complete EEG signal includes EEG signals corresponding to multiple acquisition channels. 702 in FIG. 7A may represent one electrode.

1. Establish a brain network, and fuse the brain network into an initial EEG classification model

The EEG signal is a multi-channel time sequence obtained by recording discharges of brain neurons from the scalp by a precise electronic instrument. Each channel corresponds to a location on the scalp of human brain. The channels have certain associations in three-dimensional space, and the association is helpful for the fusion of channel information. Therefore, EEG signal classification can be implemented based on a brain network. The brain network is a network used for describing functional connections of the brain, which includes nodes and connection edges. The node represents a component of the brain, that is, one acquisition channel or electrode, and the connection edge represents a connection relationship between components, that is, an association between acquisition channels or electrodes. The brain network can be represented by a channel association feature.

1-1. Establish an initial brain network

A spatial brain network refers to a brain network established based on spatial location information of the electrodes, and is used for measuring a possible physical connection between different electrodes. Apparently, neurons that are close in space are more likely to be physically connected, while neurons that are far away from each other are less likely to be physically connected. The initial brain network is established as follows:

(1) Map three-dimensional coordinates of electrodes to a two-dimensional system to obtain two-dimensional coordinates corresponding to the electrodes.

(2) Considering each electrode as a vertex of a triangle, obtain side lengths of the triangle by calculating a distance between every two vertexes.

(3) When and only when the following conditions are met, determine that a triangles and sides of the triangles exist: ① the triangle does not include any nodes except three vertexes; ② the triangle should be an acute triangle or a right triangle (that is, the triangle cannot be an obtuse triangle).

(4) Count all existing triangles, and construct a spatial brain network by using sides of each triangle as connection edges of the network, to obtain an initial adjacency matrix corresponding to the initial brain network. The dimensionality of the initial adjacency matrix is a node quantity (that is, the electrode quantity or channel quantity), and a numerical value represents a connection edge value between every two nodes. In a case that the brain network is an unweighted network, values in the initial adjacency matrix only include 0 or 1. In a case that the brain network is a weighted network, data in the initial adjacency matrix includes 0 and a reciprocal of a spatial distance between two electrodes corresponding to each connection edge.

1-2. Perform normalization processing on the initial brain network to obtain a target brain network

First, the initial adjacency matrix A and the cell matrix I are summed based on the formula $\tilde{A} = A + I$ to obtain an intermediate adjacency matrix $\tilde{A}$ . This is because the original multi-channel signal needs to be multiplied with the normalized adjacency matrix during subsequent network embedding. If the cell matrix is not added, each channel signal after the calculation dos not include information of the channel, which is not conducive to the subsequent analysis.
Then, normalization processing is performed on the initial brain network through the formula

$$\hat{A} = D^{-\frac{1}{2}}\tilde{A}D^{-\frac{1}{2}}$$

to obtain a target brain network (that is, the target channel association feature). The normalization processing can transform the initial adjacency matrix into a matrix of rows and columns in which values are 1, so that an assignment interval of the original signal is not altered during subsequent brain network fusion. The target brain network is a structural brain network used for describing physical connections or locational relationships between neurons or regions of the brain.

1-3. Fuse the target brain network into the initial EEG classification model to serve as a component of the initial EEG classification model

The target brain network is constructed based on the spatial location relationship of the acquisition channels of the EEG signal, and the target brain network is integrated into a model structure of a deep learning model to fully utilize spatial information between the acquisition channels and integrate highly connected acquisition channels, thereby improving the performance of the EEG signal classification model.
Further, if the target brain network is integrated into the initial EEG classification model, one initial EEG classification model corresponds to one acquisition device. In this way, for different samples corresponding to acquisition channels at the same location, the brain network only needs to be calculated once, which is more efficient and suitable for a brain-computer interface system that needs real-time control.

2. Perform supervised training on the initial EEG signal classification model to obtain a target EEG signal classification model

Supervised training is performed on the initial EEG signal classification model based on the training EEG signal and the corresponding training label to obtain the target EEG signal classification model.

3. Perform EEG signal classification through the target EEG signal classification model

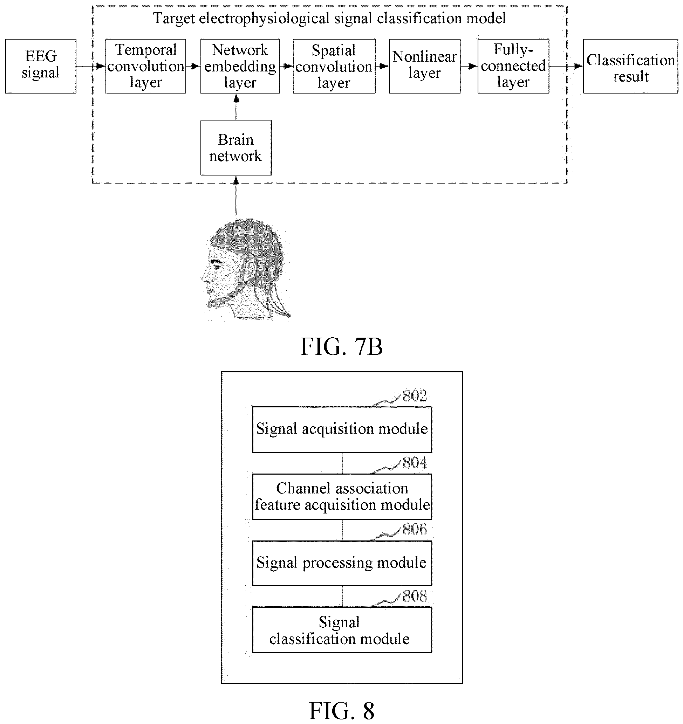

Referring to FIG. 7B, the target electrophysiological signal classification model includes a temporal convolution layer, a network embedding layer, a spatial convolution layer, a nonlinear layer, and a fully-connected layer that are sequentially connected. The network embedding layer is integrated with the target brain network (that is, the target channel association feature). After a to-be-classified EEG signal is inputted into the target EEG signal classification model, a target time feature corresponding to the to-be-classified EEG signal can be extracted through the temporal convolution layer. The target time feature is embedded into the target brain network through the network embedding layer to obtain a target embedded feature. A spatial feature corresponding to the target embedded feature is extracted through the spatial convolution layer. Nonlinear processing is performed on the target spatial feature the through the nonlinear layer to obtain a fitting feature, and classification processing is performed on the fitting feature through the fully-connected layer to obtain a classification result corresponding to the to-be-classified EEG signal. In some embodiments of the present disclosure, the fitting feature can be preferably configured as the target fitting feature.

In all embodiments of the present disclosure, end-to-end decoding of the EEG signal can be implemented without artificial feature extraction, making the model more universal. In addition, the spatial brain network is integrated into the construction of the deep learning model, and topological connections between electrodes are introduced while the original signal feature is preserved, so that multi-channel signals are better fused, thereby obtaining a more accurate EEG classification result. Moreover, the network is built based on the spatial locations of the electrodes, and signal samples with the same electrode location have the same spatial brain network, which achieves high computation efficiency and is suitable for the brain-machine interface system that needs real-time control. For example, for a user with hand disability, the computer device can acquire an EEG signal of the user in real time. Motion imagination classification is performed on the EEG signal through the target EEG signal classification model. In a case that the classification result shows that the user is currently imagining the left hand, the computer device can drive a left mechanical arm to move.

It should be understood that in addition to the application to the EEG classification task, the method for classification processing of an electrophysiological signal in this application can also be applied to other electrophysiological signal classification tasks, for example, an ECG classification task, an EMG signal classification task, or the like. For example, in a case that a user is exercising, muscle state classification can be performed on an EMG signal of the user through the target EMG signal classification model. In a case that a muscle state is muscle fatigue, prompt information is generated to prompt the user to rest in time.

It is to be understood that, steps in flowcharts of FIG. 2 to FIG. 6 are displayed in sequence based on indication of arrows, but the steps are not necessarily performed in sequence based on a sequence indicated by the arrows. Unless otherwise explicitly specified in this specification, execution of the steps is not strictly limited, and the steps may be performed in other sequences. In addition, at least some steps in FIG. 2 to FIG. 6 may include a plurality of steps or a plurality of stages, and these steps or stages are not necessarily performed at a same time instant, but may be performed at different time instants. The steps or stages are not necessarily performed in sequence, but may be performed by turn or alternately with other steps or at least part of steps or stages in other steps.

In all embodiments of the present disclosure, as shown in FIG. 8, an exemplary apparatus for classification processing of an electrophysiological signal is provided. The apparatus may include software modules or hardware modules, or include a combination of the software and hardware modules to become a part of a computer device. The apparatus specifically includes: a signal acquisition module 802, a channel association feature acquisition module 804, a signal processing module 806, and a signal classification module 808.

The signal acquisition module 802 is configured to acquire a to-be-classified electrophysiological signal collected by an acquisition device. In some embodiments of the present disclosure, the acquisition device can be preferably configured as the target acquisition device.

The channel association feature acquisition module 804 is configured to acquire a channel association feature, where the channel association feature is generated based on spatial locations of multiple acquisition channels on the acquisition device. In some embodiments of the present disclosure, the channel association feature can be preferably configured as the target channel association feature.

The signal processing module 806 is configured to extract a time feature corresponding to the to-be-classified electrophysiological signal through a electrophysiological signal classification model, and embed the channel association feature into the time feature to obtain an embedded feature. In some embodiments of the present disclosure, the time feature can be preferably configured as the target time feature, and the embedded feature can be preferably configured as the target embedded feature.

The signal classification module 808 is configured to extract a spatial feature from the obtained embedded feature through the electrophysiological signal classification model, and obtain a classification result of the electrophysiological signal based on the spatial feature. In some embodiments of the present disclosure, the spatial feature can be preferably configured as the target spatial feature.

In all embodiments of the present disclosure, the channel association feature acquisition module can be further configured to acquire the target electrophysiological signal classification model corresponding to the target acquisition device, where the target electrophysiological signal classification model is integrated with the target channel association feature corresponding to the target acquisition device, and is used for extracting the target time feature corresponding to the to-be-classified electrophysiological signal, embedding the target channel association feature into the target time feature to obtain the target embedded feature, extracting the target spatial feature corresponding to the target embedded feature, and obtaining the classification result corresponding to the to-be-classified electrophysiological signal based on the target spatial feature.

In all embodiments of the present disclosure, the channel association feature acquisition module can be further configured to determine a target classification task; acquire multiple candidate electrophysiological signal classification models corresponding to the target classification task, the multiple candidate electrophysiological signal classification models having corresponding candidate acquisition devices; and determine the target electrophysiological signal classification model corresponding to the target acquisition device from the candidate electrophysiological signal classification models.

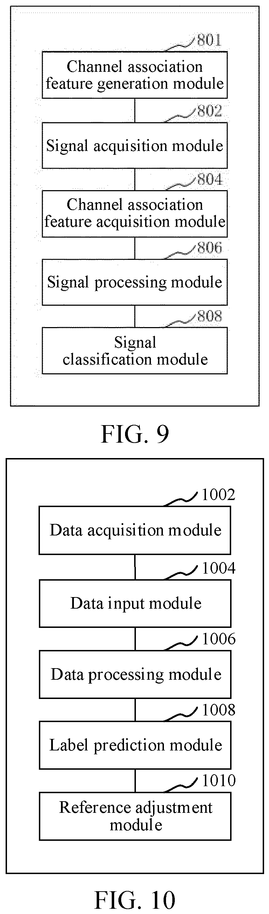

In all embodiments of the present disclosure, as shown in FIG. 9, the exemplary apparatus further includes: a channel association feature generation module 801, configured to map the spatial locations of the acquisition channels to a same plane to obtain plane locations of the acquisition channels; acquire a target quantity of acquisition channels from the acquisition channels as target acquisition channels to obtain a target quantity of target acquisition channels; generate a target channel region based on the plane locations of the target acquisition channels; associate the target acquisition channels in a case that a region shape feature corresponding to the target channel region is a preset shape feature and there is no other acquisition channel in the target channel region; and generate the target channel association feature based on the associated acquisition channels.

In all embodiments of the present disclosure, the channel association feature generation module can be further configured to use each of the acquisition channels as a region vertex to obtain multiple region vertexes, and connect the region vertexes in turn to obtain the target channel region.

In all embodiments of the present disclosure, the region shape feature can include vertex angles corresponding to the region vertexes of the target channel region. The channel association feature generation module is further configured to determine that the region shape feature corresponding to the target channel region is the preset shape feature in a case that the vertex angles are all within a reference angle range; and associate the target acquisition channels in a case that there is no other acquisition channel in the target channel region whose region shape feature is the preset shape feature.

In all embodiments of the present disclosure, the channel association feature generation module can be further configured to generate an initial channel association feature based on the associated acquisition channels; and perform normalization processing on the initial channel association feature to obtain the target channel association feature.

In all embodiments of the present disclosure, the channel association feature generation module can be further configured to generate an initial channel association matrix based on the initial channel association feature; acquire a cell matrix, and fuse the cell matrix and the initial channel association matrix to obtain an intermediate channel association matrix; acquire a diagonal matrix corresponding to the initial channel association matrix, and fuse the diagonal matrix and the intermediate channel association matrix to obtain a target channel association matrix; and obtain the target channel association feature based on the target channel association matrix.

In all embodiments of the present disclosure, the channel association feature generation module can be further configured to determine matrix dimensionality of the initial channel association matrix based on a channel quantity of the acquisition channels on the target acquisition device, set matrix values corresponding to the associated acquisition channels to a first preset threshold, and set matrix values corresponding to other acquisition channels to a second preset threshold, to obtain the initial channel association matrix. The channel association feature generation module is further configured to determine matrix dimensionality of the initial channel association matrix based on a channel quantity of the acquisition channels on the target acquisition device, determine matrix values corresponding to the associated acquisition channels based on spatial location distances between the associated acquisition channels, and set matrix values corresponding to other acquisition channels to a second preset threshold to obtain the initial channel association matrix.

In all embodiments of the present disclosure, the to-be-classified electrophysiological signal can include electrophysiological signals corresponding to the acquisition channels respectively. The signal processing module is further configured to acquire at least one temporal convolution kernel; separately extract time sub-features corresponding to electrophysiological signals based on a same temporal convolution kernel to obtain multiple time sub-features corresponding to each of the at least one temporal convolution kernel; generate an intermediate time feature based on the multiple time sub-features corresponding to the same temporal convolution kernel to obtain at least one intermediate time feature corresponding to the at least one temporal convolution kernel respectively; and obtain the target time feature based on the at least one intermediate time feature.

In all embodiments of the present disclosure, the target time feature can include multiple intermediate time features. The signal processing module is further configured to separately embed the target channel association feature into the intermediate time features to obtain initial embedded features corresponding to the intermediate time features, and obtain the target embedded feature based on the initial embedded features.

In all embodiments of the present disclosure, the channel association feature can include association sub-features corresponding to the acquisition channels respectively, and the intermediate time feature includes time sub-features corresponding to the acquisition channels respectively. The signal processing module is further configured to: in a current intermediate time feature, embed an association sub-feature corresponding to a same acquisition channel into a corresponding time sub-feature, to obtain embedded sub-features corresponding to the acquisition channels respectively; and obtain an initial embedded feature corresponding to the current intermediate time feature based on the embedded sub-features.

In all embodiments of the present disclosure, the signal classification module can be further configured to acquire at least one spatial convolution kernel; perform spatial feature extraction on the target embedded feature separately based on the at least one spatial convolution kernel to obtain at least one intermediate spatial feature corresponding to the at least one spatial convolution kernel respectively; and obtain the target spatial feature based on the at least one intermediate spatial feature.

In all embodiments of the present disclosure, the signal classification module can be further configured to perform nonlinear processing on the target spatial feature to obtain a target fitting feature; and perform classification processing on the target fitting feature to obtain the classification result.

In all embodiments of the present disclosure, as shown in FIG. 10, an exemplary apparatus for classification processing of an electrophysiological signal is provided. The apparatus may include software modules or hardware modules, or include a combination of software and hardware modules to become a part of a computer device. The apparatus specifically includes: a data acquisition module 1002, a data input module 1004, a data processing module 1006, a label prediction module 1008, and a reference adjustment module 1010.

The data acquisition module 1002 is configured to acquire a training electrophysiological signal collected by an acquisition device and a training label of the training electrophysiological signal. In some embodiments of the present disclosure, the acquisition device can be preferably configured as the target acquisition device.

The data input module 1004 is configured to input the training electrophysiological signal into an initial electrophysiological signal classification model corresponding to the acquisition device, the initial electrophysiological signal classification model including a channel association feature, the channel association feature being generated based on spatial locations of multiple acquisition channels on the acquisition device. In some embodiments of the present disclosure, the channel association feature can be preferably configured as the target channel association feature.

The data processing module 1006 is configured to extract a time feature from the training electrophysiological signal through the initial electrophysiological signal classification model, and embed the channel association feature into the time feature to obtain an embedded feature. In some embodiments of the present disclosure, the time feature can be preferably configured as the target time feature, and the embedded feature can be preferably configured as the target embedded feature.

The label prediction module 1008 is configured to extract a spatial feature from the obtained embedded feature through the initial electrophysiological signal classification model, and obtain a predicted label of the training electrophysiological signal based on the spatial feature. In some embodiments of the present disclosure, the spatial feature can be preferably configured as the target spatial feature.

The reference adjustment module 1010 is configured to adjust a model parameter of the initial electrophysiological signal classification model based on a difference between the training label and the predicted label, until a convergence condition is met, to obtain a electrophysiological signal classification model. In some embodiments of the present disclosure, the electrophysiological signal classification model can be preferably configured as the target electrophysiological signal classification model.

For a specific limitation on the apparatus for classification processing of an electrophysiological signal, refer to the limitation on the method for classification processing of an electrophysiological signal above. Details are not described herein again. Each module in the foregoing apparatus for classification processing of an electrophysiological signal may be implemented entirely or partially by software, hardware, and a combination thereof. The foregoing modules may be built in or independent of one or more processors of a computer device in a hardware form, or may be stored in a memory of the computer device in a software form, so that the processor invokes and performs an operation corresponding to each of the foregoing modules.

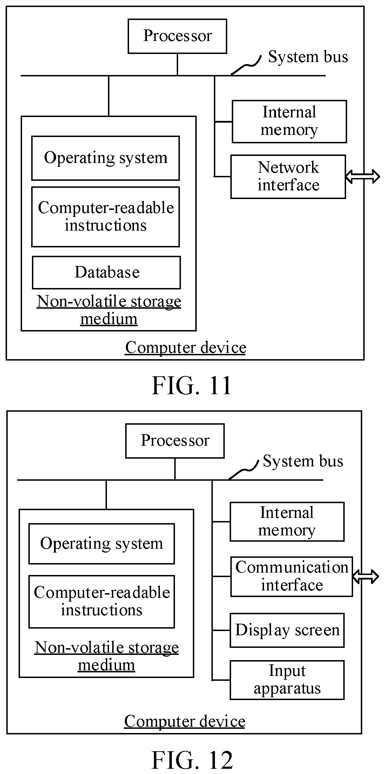

In all embodiments of the present disclosure, an exemplary computer device is provided. The computer device may be a server, and an internal structure diagram thereof may be shown in FIG. 11. The computer device comprises a processor, a memory, and a network interface that are connected by using a system bus. The processor of the terminal is configured to provide computing and control capabilities. The memory of the terminal comprises a non-volatile storage medium and an internal memory. The non-volatile storage medium stores an operating system, a computer-readable instruction, and a database. The internal memory provides an environment for running of the operating system and the computer-readable instruction in the non-volatile storage medium. The database of the computer device is configured to store data such as candidate electrophysiological signal classification models, a target electrophysiological signal classification model, and the like. The network interface of the computer device is configured to communicate with an external terminal through a network connection. The computer-readable instruction is executed by the processor to implement the method for classification processing of an electrophysiological signal.

In all embodiments of the present disclosure, an exemplary computer device is provided. The computer device may be a terminal, and an internal structure diagram thereof may be shown in FIG. 12. The computer device includes a processor, a memory, a communication interface, a display screen, and an input apparatus that are connected by using a system bus. The processor of the terminal is configured to provide computing and control capabilities. The memory of the terminal comprises a non-volatile storage medium and an internal memory. The non-volatile storage medium stores an operating system and a computer-readable instruction. The internal memory provides an environment for running of the operating system and the computer-readable instruction in the non-volatile storage medium. The communication interface of the computer device is configured to communicate with an external terminal in a wired or wireless manner, and the wireless manner can be implemented by using WIFI, an operator network, near-field communication (NFC), or other technologies. The computer-readable instruction is executed by the processor to implement the method for classification processing of an electrophysiological signal. The display screen of the computer device may be a liquid crystal display screen or an electronic ink display screen. The input apparatus of the computer device may be a touch layer covering the display screen, or may be a key, a trackball, or a touch pad disposed on a housing of the computer device, or may be an external keyboard, a touch pad, a mouse, or the like.

A person skilled in the art may understand that, the structures shown in FIG. 11 and FIG. 12 are only block diagrams of partial structures related to the solution of this application and do not limit the computer device to which the solution of this application is applied. Specifically, the computer device may include more or fewer components than those in the drawings, or some components are combined, or a different component deployment is used.

In all embodiments of the present disclosure, an exemplary computer device is further provided, including a memory and one or more processors, the memory storing computer-readable instructions, the computer-readable instructions, when executed by the one or more processors, implementing the steps in the foregoing method embodiments.

In all embodiments of the present disclosure, an exemplary computer-readable storage medium is provided, storing computer-readable instructions, the computer-readable instructions, when executed by one or more processors, implementing the steps in the foregoing method embodiments.

In all embodiments of the present disclosure, an exemplary computer program product or a computer program is provided. The computer program product or the computer program includes computer instructions, and the computer instructions are stored in a computer-readable storage medium. The one or more processors of the computer device read the computer instructions from the computer-readable storage medium, and the one or more processors execute the computer instructions, to cause the computer device to perform the steps in the method embodiments.

A person of ordinary skill in the art may understand that all or some of the procedures of the methods of the foregoing embodiments may be implemented by computer-readable instructions instructing relevant hardware. The computer-readable instructions may be stored in a non-volatile computer-readable storage medium. When the computer-readable instructions are executed, the procedures of the embodiments of the foregoing methods may be included. Any reference to a memory, a storage, a database, or another medium used in the embodiments provided in this application may include at least one of a non-volatile memory and a volatile memory. The non-volatile memory may include a read-only memory (ROM), a magnetic tape, a floppy disk, a flash memory, an optical memory, and the like. The volatile memory may include a random access memory (RAM) or an external cache. For the purpose of description instead of limitation, the RAM is available in a plurality of forms, such as a static RAM (SRAM) or a dynamic RAM (DRAM).

The technical features in the foregoing embodiments may be randomly combined. For concise description, not all possible combinations of the technical features in the embodiment are described. However, provided that combinations of the technical features do not conflict with each other, the combinations of the technical features are considered as falling within the scope recorded in this specification.

The foregoing embodiments only describe several implementations of this application specifically and in detail, but cannot be construed as a limitation to the patent scope of this application. It should be noted that a person of ordinary skill in the art may make various changes and improvements without departing from the ideas of this application, which shall all fall within the protection scope of this application. Therefore, the protection scope of this patent application is subject to the protection scope of the appended claims.

**Claims**

1. A method for classification processing of an electrophysiological signal, performed by a computer device, the method comprising:

   acquiring a to-be-classified electrophysiological signal collected by an acquisition device;
   acquiring a channel association feature, the channel association feature being generated based on spatial locations of multiple acquisition channels on the acquisition device;
   extracting a time feature from the collected electrophysiological signal, and embedding the channel association feature into the time feature to obtain an embedded feature; and
   extracting a spatial feature from the obtained embedded feature, and obtaining a classification result of the electrophysiological signal based on the spatial feature.

2. The method according to claim 1, wherein the acquiring a channel association feature comprises:

   acquiring an electrophysiological signal classification model corresponding to the target acquisition device, wherein
   the electrophysiological signal classification model is integrated with the channel association feature, and is configured to extract the time feature from the collected electrophysiological signal, embedding the channel association feature into the time feature to obtain the embedded feature, extracting the spatial feature from the obtained embedded feature, and obtaining the classification result of the electrophysiological signal based on the spatial feature.

3. The method according to claim 2, wherein the acquiring an electrophysiological signal classification model comprises:

   determining a classification task;
   acquiring multiple candidate electrophysiological signal classification models corresponding to the classification task, the candidate electrophysiological signal classification models having corresponding candidate acquisition devices; and
   determining the electrophysiological signal classification model corresponding to the acquisition device from the candidate electrophysiological signal classification models.

4. The method according to claim 1, wherein the channel association feature is generated through the following steps:

   mapping spatial locations of acquisition channels to a same plane to obtain plane locations of the acquisition channels;
   acquiring a quantity of acquisition channels from the acquisition channels;
   generating a channel region based on the plane locations of the quantity of the acquisition channels;
   associating the quantity of the acquisition channels in a case that a region shape feature of the channel region is a preset shape feature and there is no other acquisition channel in the channel region ; and
   generating the channel association feature based on the associated acquisition channels.

5. The method according to claim 4, wherein the region shape feature comprises vertex angles corresponding to region vertexes of the channel region, and the associating the quantity of the acquisition channels in a case that a region shape feature of the channel region is a preset shape feature and there is no other acquisition channel in the channel region comprises:

   determining that the region shape feature of the channel region is the preset shape feature in a case that the vertex angles are all within a reference angle range; and
   associating the quantity of the acquisition channels based on connection relationships between the region vertexes in a case that there is no other acquisition channel in the channel region of which the region shape feature is the preset shape feature.

6. The method according to claim 4, wherein the generating the channel association feature based on the associated acquisition channels comprises:

   generating an initial channel association feature based on the associated acquisition channels; and
   performing normalization processing on the initial channel association feature to obtain the channel association feature.

7. The method according to claim 6, wherein the performing normalization processing on the initial channel association feature to obtain the channel association feature comprises:

generating an initial channel association matrix based on the initial channel association feature;
acquiring a cell matrix, and fusing the cell matrix and the initial channel association matrix to obtain an intermediate channel association matrix;
acquiring a diagonal matrix corresponding to the initial channel association matrix, and fusing the diagonal matrix and the intermediate channel association matrix to obtain a channel association matrix; and
obtaining the channel association feature based on the channel association matrix.

8. The method according to claim 7, wherein the generating an initial channel association matrix based on the initial channel association feature comprises any one of the following manners:

determining matrix dimensionality of the initial channel association matrix based on a channel quantity of the acquisition channels on the acquisition device, setting matrix values corresponding to the associated acquisition channels to a first preset threshold, and setting matrix values corresponding to other acquisition channels to a second preset threshold, to obtain the initial channel association matrix; or
determining matrix dimensionality of the initial channel association matrix based on a channel quantity of the acquisition channels on the acquisition device, determining matrix values corresponding to the associated acquisition channels based on spatial location distances between the associated acquisition channels, and setting matrix values corresponding to other acquisition channels to a second preset threshold, to obtain the initial channel association matrix.

9. The method according to claim 1, wherein the to-be-classified electrophysiological signal comprises electrophysiological signals corresponding to the acquisition channels respectively, and the extracting a time feature from the collected electrophysiological signal comprises:

acquiring at least one temporal convolution kernel;
separately extracting time sub-features corresponding to the electrophysiological signals based on a same temporal convolution kernel to obtain multiple time sub-features corresponding to each of the at least one temporal convolution kernel;
generating an intermediate time feature based on the multiple time sub-features corresponding to the same temporal convolution kernel to obtain at least one intermediate time feature corresponding to the at least one temporal convolution kernel respectively; and
obtaining the time feature based on the at least one intermediate time feature.

10. The method according to claim 1, wherein the time feature comprises multiple intermediate time features, and the embedding the channel association feature into the time feature to obtain an embedded feature comprises:

separately embedding the channel association feature into the intermediate time features to obtain initial embedded features corresponding to the intermediate time features; and
obtaining the embedded feature based on the intermediate initial embedded features.

11. The method according to claim 10, wherein the channel association feature comprises association sub-features corresponding to the acquisition channels respectively, and the intermediate time features comprise time sub-features corresponding to the acquisition channels respectively; and
the separately embedding the channel association feature into the intermediate time features to obtain initial embedded features corresponding to the intermediate time features comprises:

in a current intermediate time feature, embedding an association sub-feature corresponding to a same acquisition channel into a corresponding time sub-feature, to obtain embedded sub-features corresponding to the acquisition channels respectively; and
obtaining an initial embedded feature corresponding to the current intermediate time feature based on the embedded sub-features.

12. The method according to claim 1, wherein the extracting a spatial feature from the obtained embedded feature comprises:

acquiring at least one spatial convolution kernel;
performing spatial feature extraction on the embedded feature based on the at least one spatial convolution kernel to obtain at least one intermediate spatial feature corresponding to the at least one spatial convolution kernel respectively; and
obtaining the spatial feature based on the at least one intermediate spatial feature.

13. The method according to claim 1, wherein the obtaining a classification result of the electrophysiological signal based on the spatial feature comprises:

performing nonlinear processing on the spatial feature to obtain a fitting feature; and
performing classification processing on the fitting feature to obtain the classification result.

14. A method for classification processing of an electrophysiological signal, performed by a computer device, the method comprising:

acquiring a training electrophysiological signal collected by an acquisition device and a training label of the training electrophysiological signal;
inputting the training electrophysiological signal into an initial electrophysiological signal classification model corresponding to the acquisition device, the initial electrophysiological signal classification model comprising a channel association feature, the channel association feature being generated based on spatial locations of multiple acquisition channels on the acquisition device;
extracting a time feature from the training electrophysiological signal through the initial electrophysiological signal classification model, and embedding the channel association feature into the time feature to obtain an embedded feature;
extracting a spatial feature from the obtained embedded feature through the initial electrophysiological signal classification model, and obtaining a predicted label of the training electrophysiological signal based on the spatial feature; and
adjusting a model parameter of the initial electrophysiological signal classification model based on a difference between the training label and the predicted label, until a convergence condition is met, to obtain a electrophysiological signal classification model.

15. An apparatus for classification processing of an electrophysiological signal, comprising:

a signal acquisition module, configured to acquire a to-be-classified electrophysiological signal collected by an acquisition device;
a channel association feature acquisition module, configured to acquire a channel association feature, the channel association feature being generated based on spatial locations of multiple acquisition channels on the acquisition device;
a signal processing module, configured to extract a time feature from the collected electrophysiological signal, and embed the channel association feature into the time feature to obtain an embedded feature; and
a signal classification module, configured to extract a spatial feature from the obtained embedded feature, and obtain a classification result of the electrophysiological signal based on the spatial feature.

16. An apparatus for classification processing of an electrophysiological signal, comprising:

a data acquisition module, configured to acquire a training electrophysiological signal collected by an acquisition device and a training label of the training electrophysiological signal;
a data input module, configured to input the training electrophysiological signal into an initial electrophysiological signal classification model corresponding to the acquisition device, the initial electrophysiological signal classification model comprising a channel association feature, the channel association feature being generated based on spatial locations of multiple acquisition channels on the acquisition device;
a data processing module, configured to extract a time feature from the training electrophysiological signal through the initial electrophysiological signal classification model, and embed the channel association feature into the time feature to obtain an embedded feature;
a label prediction module, configured to extract a spatial feature from the obtained embedded feature through the initial electrophysiological signal classification model, and obtain a predicted label of the training electrophysiological signal based on the spatial feature; and

a reference adjustment module, configured to adjust a model parameter of the initial electrophysiological signal classification model based on a difference between the training label and the predicted label, until a convergence condition is met, to obtain an electrophysiological signal classification model.

17. A computer device, comprising a memory and one or more processors, the memory storing computer-readable instructions, the one or more processors, when executing the computer-readable instructions, implementing the steps of the method according to any one of claims 1 to 13 or claim 14.

18. One or more non-volatile computer-readable storage medium, storing computer-readable instructions, the computer-readable instructions, when executed by one or more processors, implementing the steps of the method according to any one of claims 1 to 13 or claim 14.

19. A computer program product, comprising computer-readable instructions, the computer-readable instructions, when executed by one or more processors, implementing the steps of the method according to any one of claims 1 to 13 and claim 14.

FIG. 1

_S202

Acquire a to-be-classified electrophysiological signal collected by an acquisition device

_S204

Acquire a channel association feature, the channel association feature being generated based on spatial locations of multiple acquisition channels on the acquisition device

_S206

Extract a time feature from the collected electrophysiological signal, and embed the channel association feature into the time feature to obtain an embedded feature

_S208

Extract a spatial feature from the obtained embedded feature, and obtain a classification result of the electrophysiological signal based on the spatial feature

FIG. 2

S302

Map spatial locations of the acquisition channels to a same plane to obtain plane locations of the acquisition channels

S304

Acquire a quantity of acquisition channels from the acquisition channels

S306

Generate a channel region based on the plane locations of the quantity of the acquisition channels

S308

Associate the quantity of the acquisition channels in a case that a region shape feature of the channel region is a preset shape feature and there is no other acquisition channel in the channel region

S310

Generate the channel association feature based on the associated acquisition channels

FIG. 3A

FIG. 3B

```
                                                      ⤳ S302
┌─────────────────────────────────────────────────────┐
│                                                     │
│  Map spatial locations of the acquisition channels to a same  │
│  plane to obtain plane locations of the acquisition channels  │
│                                                     │
└─────────────────────────────────────────────────────┘
                          │
                          ▼               ⤳ S304
┌─────────────────────────────────────────────────────┐
│                                                     │
│  Acquire a quantity of acquisition channels from the acquisition  │
│                      channels                       │
│                                                     │
└─────────────────────────────────────────────────────┘
                          │
                          ▼               ⤳ S306
┌─────────────────────────────────────────────────────┐
│                                                     │
│  Generate a channel region based on the plane locations of the  │
│            quantity of the acquisition channels     │
│                                                     │
└─────────────────────────────────────────────────────┘
                          │
                          ▼               ⤳ S308
┌─────────────────────────────────────────────────────┐
│  Associate the quantity of the acquisition channels in a case that  │
│  a region shape feature of the channel region is a preset shape  │
│  feature and there is no other acquisition channel in the channel  │
│                        region                       │
└─────────────────────────────────────────────────────┘
                          │
                          ▼               ⤳ S402
┌─────────────────────────────────────────────────────┐
│                                                     │
│  Generate an initial channel association feature based on the  │
│            associated acquisition channels          │
│                                                     │
└─────────────────────────────────────────────────────┘
                          │
                          ▼               ⤳ S404
┌─────────────────────────────────────────────────────┐
│                                                     │
│  Perform normalization processing on the initial channel  │
│  association feature to obtain the channel association feature  │
│                                                     │
└─────────────────────────────────────────────────────┘
```

FIG. 4

Map spatial locations of the acquisition channels to a same plane to obtain plane locations of the acquisition channels ⟋S302

↓

Acquire a quantity of acquisition channels from the acquisition channels ⟋S304

↓

Generate a channel region based on the plane locations of the quantity of the acquisition channels ⟋S306

↓

Associate the quantity of the acquisition channels in a case that a region shape feature of the channel region is a preset shape feature and there is no other acquisition channel in the channel region ⟋S308

↓

Generate an initial channel association feature based on the associated acquisition channels ⟋S402

Generate an initial channel association matrix based on the initial channel association feature ⟋S502

↓

Acquire a cell matrix, and fuse the cell matrix and the initial channel association matrix to obtain an intermediate channel association matrix ⟋S504

↓

Acquire a diagonal matrix corresponding to the initial channel association matrix, and fuse the diagonal matrix and the intermediate channel association matrix to obtain a channel association matrix ⟋S506

↓

Obtain the channel association feature based on the channel association matrix ⟋S508

FIG. 5

_◯_ S602

Acquire a training electrophysiological signal collected by an acquisition device and a training label of the training electrophysiological signal

↓ _◯_ S604

Input the training electrophysiological signal into an initial electrophysiological signal classification model corresponding to the acquisition device, the initial electrophysiological signal classification model including a channel association feature, the channel association feature being generated based on spatial locations of multiple acquisition channels on the acquisition device

↓ _◯_ S606

Extract a time feature from the training electrophysiological signal through the initial electrophysiological signal classification model, and embed the channel association feature into the time feature to obtain an embedded feature

↓ _◯_ S608

Extract a spatial feature from the obtained embedded feature through the initial electrophysiological signal classification model, and obtain a predicted label of the training electrophysiological signal based on the spatial feature

↓ _◯_ S610

Adjust a model parameter of the initial electrophysiological signal classification model based on a difference between the training label and the predicted label, until a convergence condition is met, to obtain a electrophysiological signal classification model

FIG. 6

EEG signal

FIG. 7A

FIG. 7B

FIG. 8

Channel association
feature generation module /801

Signal acquisition module /802

Channel association
feature acquisition module /804

Signal processing module /806

Signal
classification module /808

FIG. 9

Data acquisition module /1002

Data input module /1004

Data processing module /1006

Label prediction module /1008

Reference adjustment
module /1010

FIG. 10

Processor

System bus

Internal memory

Network interface

Operating system

Computer-readable instructions

Database

Non-volatile storage medium

Computer device

FIG. 11

Processor

System bus

Internal memory

Communication interface

Display screen

Input apparatus

Operating system

Computer-readable instructions

Non-volatile storage medium

Computer device

FIG. 12

## INTERNATIONAL SEARCH REPORT

| | |
|---|---|
| International application No. | |
| **PCT/CN2022/078970** | |

**A. CLASSIFICATION OF SUBJECT MATTER**

G06K 9/00(2022.01)i

According to International Patent Classification (IPC) or to both national classification and IPC

**B. FIELDS SEARCHED**

Minimum documentation searched (classification system followed by classification symbols)

G06K9/

Documentation searched other than minimum documentation to the extent that such documents are included in the fields searched

Electronic data base consulted during the international search (name of data base and, where practicable, search terms used)

WPABSC; CNTXT; CNABS; ENTXT; DWPI; VEN; CNKI; IEEE: 生理信号, 生理电, 脑电, 肌电, 心电, 眼电, 时间, 空间, 时序, 时域, 空域, 位置, 卷积, 卷积神经网络, 循环, 长短时记忆, 通道, 投影, 二维, 平面, 区域, 形状, physiological signal, physiological electricity, electroencephalogram, electromyography, electrocardiograph, electro oculogram, EEG, EMG, ECG, EOG, time, space, spatial, time sequence, time series, time domain, spatial domain, position, location, convolution, CNN, recurrent, RNN, long short term memory, LSTM, channel, project, two dimension, 2D, plane, area, shape

**C. DOCUMENTS CONSIDERED TO BE RELEVANT**

| Category* | Citation of document, with indication, where appropriate, of the relevant passages | Relevant to claim No. |
|---|---|---|
| PX | CN 112597986 A (TENCENT TECHNOLOGY SHENZHEN CO., LTD.) 02 April 2021 (2021-04-02) claims 1-15, and description, paragraphs 7-9 and 176 | 1-19 |
| X | CN 107961007 A (CHONGQING UNIVERSITY OF POSTS AND TELECOMMUNICATIONS) 27 April 2018 (2018-04-27) description, paragraphs 49-75, and figures 1-2 | 1-3, 9-19 |
| A | CN 109924990 A (LANZHOU UNIVERSITY) 25 June 2019 (2019-06-25) entire document | 1-19 |
| A | CN 111163690 A (SHENZHEN INSTITUTES OF ADVANCED TECHNOLOGY) 15 May 2020 (2020-05-15) entire document | 1-19 |
| A | CN 111317468 A (TENCENT TECHNOLOGY SHENZHEN CO., LTD.) 23 June 2020 (2020-06-23) entire document | 1-19 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **12 May 2022** | **18 May 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/078970** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2021014150 A1 (UNIV OXFORD INNOVATION LTD.) 28 January 2021 (2021-01-28) entire document | 1-19 |
| X | GAO, Zhongke et al. "A Deep Learning Method for Improving the Classification Accuracy of SSMVEP-Based BCI" *IEEE TRANSACTIONS ON CIRCUITS AND SYSTEMS -II: EXPRESS BRIEFS,* Vol. 67, No. 12, 26 March 2020 (2020-03-26), pp. 3447-3451 | 1-3, 9-19 |

Form PCT/ISA/210 (second sheet) (January 2015)

EP 4 303 759 A1

INTERNATIONAL SEARCH REPORT
Information on patent family members

International application No.

PCT/CN2022/078970

| Patent document cited in search report | | | Publication date (day/month/year) | Patent family member(s) | | | Publication date (day/month/year) |
|---|---|---|---|---|---|---|---|
| CN | 112597986 | A | 02 April 2021 | CN | 112597986 | B | 08 June 2021 |
| CN | 107961007 | A | 27 April 2018 | | None | | |
| CN | 109924990 | A | 25 June 2019 | | None | | |
| CN | 111163690 | A | 15 May 2020 | JP | 2021526063 | A | 30 September 2021 |
| | | | | EP | 3847958 | A1 | 14 July 2021 |
| | | | | KR | 20210037614 | A | 06 April 2021 |
| | | | | WO | 2020047750 | A1 | 12 March 2020 |
| | | | | EP | 3847958 | A4 | 08 September 2021 |
| CN | 111317468 | A | 23 June 2020 | | None | | |
| WO | 2021014150 | A1 | 28 January 2021 | GB | 201910657 | D0 | 11 September 2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021102464941